(19)

Europäisches Patentamt

European Patent Office

Office européen des brevets

(11) **EP 4 125 091 B1**

(12) **EUROPEAN PATENT SPECIFICATION**

(45) Date of publication and mention
of the grant of the patent:
**18.02.2026 Bulletin 2026/08**

(21) Application number: **22186855.7**

(22) Date of filing: **26.07.2022**

(51) International Patent Classification (IPC):
**G16B 50/10** (2019.01)    **G16H 70/00** (2018.01)

(52) Cooperative Patent Classification (CPC):
**G16B 50/10; G16H 70/00;** Y02A 90/10

(54) **METHOD AND SYSTEM FOR ANNOTATION AND CLASSIFICATION OF BIOMEDICAL TEXT HAVING BACTERIAL ASSOCIATIONS**

VERFAHREN UND SYSTEM ZUR ANNOTATION UND KLASSIFIZIERUNG VON BIOMEDIZINISCHEN TEXTEN, DIE BAKTERIELLE ASSOZIATIONEN AUFWEISEN

PROCÉDÉ ET SYSTÈME D'ANNOTATION ET DE CLASSIFICATION DE TEXTE BIOMÉDICAL AYANT DES ASSOCIATIONS BACTÉRIENNES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.07.2021 IN 202121033646**

(43) Date of publication of application:
**01.02.2023 Bulletin 2023/05**

(73) Proprietor: **Tata Consultancy Services Limited Maharashtra (IN)**

(72) Inventors:
• **BHUSAN, Kuntal Kumar**
**411013 Pune, Maharashtra (IN)**
• **MANDE, Sharmila Shekhar**
**411013 Pune, Maharashtra (IN)**
• **POKHREL, Vatsala**
**411013 Pune, Maharashtra (IN)**
• **SRIVASTAVA, Divyanshu**
**411013 Pune, Maharashtra (IN)**
• **DAS BAKSI, Krishanu**
**411013 Pune, Maharashtra (IN)**

(74) Representative: **Goddar, Heinz J.**
**Boehmert & Boehmert**
**Anwaltspartnerschaft mbB**
**Pettenkoferstrasse 22**
**80336 München (DE)**

(56) References cited:
• **SRIVASTAVA DIVYANSHU ET AL: ""EviMass": A Literature Evidence-Based Miner for Human Microbial Associations", FRONTIERS IN GENETICS, vol. 10, 849, 13 September 2019 (2019-09-13), pages 1 - 10, XP093005265, DOI: 10.3389/fgene.2019.00849**
• **SRIVASTAVA DIVYANSHU ET AL: "'EviMass' user manual Module 1 Module 2 Module 3", FRONTIERS IN GENETICS, 13 September 2019 (2019-09-13), pages 1 - 20, XP093006697, Retrieved from the Internet <URL:https://www. frontiersin.org/articles/10.3389/ fgene.2019.00849/full#supplementary-material> [retrieved on 20221209], DOI: https://doi.org/ 10.3389/fgene.2019.00849**
• **POKHREL VATSALA ET AL: "Utilizing domain-based features to improve classification accuracy of biomedical text having bacterial associations", 2021 IEEE INTERNATIONAL CONFERENCE ON BIOINFORMATICS AND BIOMEDICINE (BIBM), IEEE, 9 December 2021 (2021-12-09), pages 2840 - 2847, XP034066942, DOI: 10.1109/BIBM52615.2021.9669294**
• **KUNTAL BHUSAN K ET AL: "'NetShift': a methodology for understanding 'driver microbes' from healthy and disease microbiome datasets", THE ISME JOURNAL, NATURE PUBLISHING GROUP UK, LONDON, vol. 13, no. 2, 4 October 2018 (2018-10-04), pages 442 - 454, XP036673997, ISSN: 1751-7362, [retrieved on 20181004], DOI: 10.1038/S41396-018-0291-X**

**(Cont. next page)**

- LO CHIEH ET AL: "MPLasso: Inferring microbial association networks using prior microbial knowledge", PLOS COMPUTATIONAL BIOLOGY, vol. 13, no. 12, 27 December 2017 (2017-12-27), US, pages 1 - 20, XP093005264, ISSN: 1553-734X, DOI: 10.1371/journal.pcbi.1005915
- LIM KUN MING KENNETH ET AL: "@MInter: automated text-mining of microbial interactions", BIOINFORMATICS, vol. 32, no. 19, 16 June 2016 (2016-06-16), GB, pages 2981 - 2987, XP093005268, ISSN: 1367-4803, DOI: 10.1093/bioinformatics/btw357
- PARK YESOL ET AL: "Discovering microbe-disease associations from the literature using a hierarchical long short-term memory network and an ensemble parser model", SCIENTIFIC REPORTS, vol. 11, no. 1, 24 February 2021 (2021-02-24), XP093005872, DOI: 10.1038/s41598-021-83966-8

**Description**

CROSS-REFERENCE TO RELATED APPLICATIONS AND PRIORITY

The present application claims priority to Indian application no. 202121033646, filed on 27 July 2.

TECHNICAL FIELD

The disclosure herein generally relates to the field of annotation of biomedical text, and, more particularly, to a method and system for annotation and classification of biomedical text having bacterial associations.

BACKGROUND

The microbiome is composed of a diverse group of microorganisms such as bacteria, fungi, protozoa and viruses. These microorganisms affect the environment where they reside (humans, rhizosphere, marine ecosystem, etc.). With the gradual increase in understanding of the role of microbiome, it has become important to catalogue the elixir of this information in easily accessible form. The biggest examples of such digital resources are the Human Microbiome Project and the integrative Human Microbiome Project (Integrative HMP (iHMP)), which have been helping researchers in gaining further knowledge based on the existing source of information. Other important projects like Global Ocean Microbiome, the Earth microbiome project and several projects cataloguing information on plant microbiome have also contributed significantly in enriching the knowledge.

A major component of microbiome is composed of bacterial communities. In order to understand how bacterial groups function in an environment, it is necessary to not just focus on functions of individual bacterium, but also on the function of the entire bacterial community present in that environment. In other words, apart from knowing bacterial diversity (along with their abundances), it is important to understand how they interact or communicate with each other in their respective environments. One of the important components of such information pertains to bacterial community structure in term of bacterial association networks. These associations can be obtained from microbiome studies by identifying correlated patterns of bacterial groups based on their abundances. However, in many cases, correlations may give a false indication of a bacterial association and always needs to be backed up by an experimental evidence. Freely available biomedical literature (e.g. PubMed) is the best sources for obtaining information for obtaining such experimentally validated bacterial associations.

Existing methods of predicting bacterial associations are mostly based on correlation of observed count data from microbiome studies. Although these methods provide a good list of candidate associations, they are prone to report high false positives. These false positives are mostly the set of bacterial associations which do not exist or have not been experimentally verified. Extraction of bacterial associations from biomedical text in scientific literature (e.g. PubMed) can be used as a source for extracting true bacterial associations as well as eliminate the false positive candidate associations obtained from a count data. Existing methods available for extracting bacterial association from biomedical literature are mostly based on generic text mining methods with limited accuracy.

SRIVASTAVA DIVYANSHU ET AL: "EviMass": A Literature Evidence-Based Miner for Human Microbial Associations" discloses the importance of understanding microbe-microbe as well as microbe-disease associations is one of the key thrust areas in human microbiome research. High throughput metagenomic and transcriptomic projects have fueled discovery of a number of new microbial associations. Consequently, a plethora of information is being added routinely to biomedical literature, thereby contributing toward enhancing our knowledge on microbial associations. In this communication, present a tool called "EviMass" (Evidence based mining of human Microbial Associations), which can assist biologists to validate their predicted hypotheses from new microbiome studies. Users can interactively query the processed back-end database for microbe-microbe and disease-microbe associations. The EviMass tool can also be used to upload microbial association networks generated from a human "disease-control" microbiome study and validate the associations from biomedical literature. Additionally, a list of differentially abundant microbes for the corresponding disease can be queried in the tool for reported evidences. The results are presented as graphical plots, tabulated summary, and other evidence statistics. EviMass is a comprehensive platform and is expected to enable microbiome researchers not only in mining microbial associations, but also enriching a new research hypothesis.

SUMMARY

Embodiments of the present disclosure present technological improvements as solutions to one or more of the above-mentioned technical problems recognized by the inventors in conventional systems. In one embodiment, a system for annotation and classification of biomedical text having bacterial associations is provided. The invention is set out in the appended claim 10.

In another aspect, a method for annotation and classification of biomedical text having bacterial associations is provided. The invention is set out in the appended set of claims 1-9 and 12.

In yet another aspect, there are provided one or more non-transitory machine-readable information storage mediums comprising one or more instructions which when executed by one or more hardware processors cause annotation and classification of biomedical text having bacterial associations. The invention is set out in the appended claim 11.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 is a network diagram of a system for annotation and classification of biomedical text having bacterial associations according to some embodiments of the present disclosure.

FIG. 2 is a schematic diagram showing operation of classifiers used in the system of FIG. 1 according to some embodiment of the present disclosure.

FIG. 3A-3B is a flowchart illustrating the steps involved in a method for annotation and classification of biomedical text having bacterial associations according to some embodiments of the present disclosure.

FIG. 4 is a flowchart illustrating steps involved in the identification of bacterial biomarkers of a disease according to some embodiments of the present disclosure.

FIG. 5 is a flowchart illustrating steps involved in designing a probiotic cocktail for the treatment of the disease according to some embodiments of the present disclosure.

FIG. 6 shows a workflow for the creation of three dictionaries according to some embodiments of the present disclosure.

FIG. 7 shows a simplistic flowchart of a method for annotation of biomedical text according to some embodiments of the present disclosure.

FIG. 8A shows a t-SNE plot generated using Bag of words algorithm according to some embodiment of the present disclosure.

FIG. 8B shows a t-SNE plot generated using term frequency-inverse document frequency (TF-iDF) algorithm according to some embodiment of the present disclosure.

FIG. 8C shows a t-SNE plot generated using a set of domain features respectively according to some embodiment of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

GLOSSARY - TERMS USED IN THE EMBODIMENTS

The term "microbiome" refers to the collection of micro-organisms like bacteria, archaea, lower and higher eukaryotes, and viruses etc. that live together in a particular ecological niche as a community.

The term "pathogen" refers to any organism that can cause disease in a host.

The term "Metagenomics" refers to a culture independent genomic analysis (including structure and function of entire or a part of nucleic acid sequences) of an assemblage of microorganisms recovered directly from environmental samples.

The term "Signaling molecules" refer to a molecular messenger in which the molecule is specifically involved in transmitting information between cells. Such molecules are released from the cell sending the signal, cross over the gap between cells by diffusion, and interact with specific receptors in another cell, triggering a response in that cell by activating a series of enzyme-controlled reactions which lead to changes inside the cell.

The term "Secondary metabolites" refers to compounds that are not required for the growth or reproduction of an organism but are produced to confer a selective advantage to the organism. E.g. antibiotics, bacteriocins, etc.

The term "Bacteriocins" refers to ribosomally synthesized antimicrobial peptides produced by bacteria, which can kill or inhibit bacterial strains closely-related or non-related to produced bacteria, but do not harm the bacteria themselves by specific immunity proteins.

The term "Toxin" refers to poisonous substance produced by a biological organism such as a microbe, animal or plant.

The terms "Anti-microbial compounds" or "AMPs" refer to short and generally positively charged peptides found in a wide variety of life forms from microorganisms to humans, having the ability to kill microbial pathogens directly, or indirectly by modulating the host defense systems.

The term "Siderophores" refers to secondary metabolites that scavenge iron from environmental stocks and deliver it to cells via specific receptor.

The term "Polyketides" refers to structurally diverse secondary metabolites, including those with antibiotic activity or toxins produced by eukaryotic cells and bacteria.

The term "Quorum Sensing" refers to a process of cell-cell communication that allows bacteria to share information about cell density and adjust gene expression accordingly.

The term "Biofilm" refers to clusters of microorganisms that stick to non-biological surfaces, such as rocks in a stream, as well as to biological surfaces like roots of plants and epithelium of animals.

The term "Auto-inducers" refers to a signaling molecule produced and used by bacteria participating in quorum sensing, that is, in cell-cell communication to coordinate community-wide regulation of processes such as biofilm formation, virulence, and bioluminescence in populations of bacteria. Such communication can occur both within and between different species of bacteria.

The terms "microbial Volatile Organic Compounds" or "mVOCs" refer to secondary metabolites produced by soil and plant-associated microorganisms which are typically small, odorous compounds with low molecular mass, high vapor pressure, low boiling point, and a lipophilic moiety. These properties facilitate evaporation and diffusion aboveground and belowground through gas- and water-filled pores in soil and rhizosphere environments.

The term "Rhizospheres" refers to the soil zone around the roots in which microbial biomass is impacted by the presence of plant roots.

The term "Secretion systems" refers to protein complexes involved in the transport of proteins from the cytoplasm into other compartments of the cell, the environment, and/or other bacteria or eukaryotic cells.

The term "Naive Bayes classifier" refers to a simple machine learning algorithm that utilizes Bayes rule together with a strong assumption that the input features are conditionally independent, given the output class. Naive Bayes classifier provides a mechanism for using the information in sample data to estimate the posterior probability $P(y \mid x)$ of each class output class y, given features x.

The term "Logistic Regression" refers to a mechanism for constructing a mathematical model or a machine learning algorithm in form of an equation that best predicts the probability of a value of the output class (e.g. the expected category of classification) as a function of the feature variables pertaining to the input data.

The terms "Support vector machines" or "SVMs" refer to particular linear classifiers which are based on the margin maximization principle. They essentially try to find the optimal hyperplane that can separate the input features according to their classes. The SVM classifier often accomplishes the classification task using several types of linear or non-linear transformation functions (also called kernels) which embed the input features, in a higher dimensional space, where a linear hyperplane separates the data into two categories.

The term "Random Forest" refers to is an ensemble learning technique, which uses decision trees as the base classifier. Each decision tree is constructed from a bootstrap sample from the original dataset. To further diversify the classifiers, at each branch in the tree, the decision of which feature to split on is restricted to a random subset of size n, from the full feature set. The random subset is chosen anew for each branching point. n is suggested to be log2(N + 1), where N is the size of the whole feature set.

The terms "Bag-of-words or BOW" refer to a method where the frequency of occurrence of each word (or a subset of most frequent words) in the constituent text of a text corpus is used as a feature for training a classifier. The 'CountVectorizer' function of 'sklearn (available in scikit-learn 1.1.1)' module in Python 3 was used for calculation of the BOW feature vector in this invention.

The terms "TF-iDF or TD-IDF" or "term frequency-inverse document frequency" refer to a term weighting scheme commonly used to represent textual documents as vectors (for purposes of classification, clustering, visualization, retrieval, etc.). In other words, the method assigns a weight to each word based on its occurrence frequency in the input text with respect to the entire text corpus (set of all the texts). With respect to text mining, TF-IDF of a term in a document belonging to a corpus is given as the product (or multiplication) of Term Frequency (TF) of the term and Inverse Document Frequency (IDF) of the term. Term Frequency (TF) of a term in a document is the ratio of count of the term in the document to the number of words in the document. The Inverse Document Frequency (IDF) of the 'term' is given as the ratio of the total number of documents in the corpus to the number of documents containing the term. The 'TfidfVectorizer' function of 'sklearn (available in scikit-learn 1.1.1)' module in Python 3 was used for calculation of the TF-IDF feature vector in this invention.

The terms "precision" and "recall" are evaluation metrics used to measure the efficiency of a classification task. While precision measures what fraction of the predicted positives are actually positives, recall measures what fraction of the actual positives are predicted as positive by the method.

The terms "F1 Score" or "F1 measure" is defined as the harmonic mean of precision and recall. It is used to measure the accuracy of a test as well as for comparison of performance of the outputs of multiple classifiers.

The term "Confusion matrix" refers to the matrix which summarizes the classification performance of a classifier with respect to some test data. It is a two-dimensional matrix, indexed in one dimension by the true class of an object and in the other by the class that the classifier assigns.

The term "Bootstrap sampling" refers to a process for creating a distribution of datasets out of a single dataset by randomly selecting a predefined subset of samples.

The terms "Classification tree or decision tree" refer to machine-learning methods for constructing prediction models from data. The models are obtained by recursively partitioning the data space and fitting a simple prediction model within each partition. As a result, the partitioning can be represented graphically as a decision tree.

The present disclosure provides a method and system for annotation and classification of biomedical text having bacterial associations. The disclosed method is microbiome specific method for extraction of information from biomedical text which provides an improvement in accuracy of the reported bacterial associations. The present disclosure uses a unique set of domain features to accurately identify bacterial associations from the biomedical text. The disclosure further provides a method to use the set of domain features to improve a microbiome crowd sourcing setup and create a refined microbial association network.

According to an embodiment of the disclosure, the system 100 is also configured to generate a refined bacterial association network corresponding to a disease or healthy state, which can be used for an improved understanding of the bacterial community structure and design therapeutic interventions. One of the ways to achieve this is by computing local and global graph properties and further comparing the values of the graph properties between the healthy and disease state. The global graph properties like density, cluster coefficient and average path length can be utilized to gather insights on the overall organization of the network and subsequently enables assessment of its modularity. The density value can be used as an indicator to understand the cross talk between the resident bacteria which are represented in the network. A bacterial network with a higher number of independent units of associated bacteria is expected to have a higher clustering coefficient value. Further, the average path length value provides a measure of the compactness of the bacterial community structure. Various local graph properties like degree, between-ness centrality and core-ness centrality can then be used on the identified refined bacterial association network to understand the individual node (or bacterium) level changes. The edge weights available in the refined association network can help in better estimation of node centralities. One can also use the edge weights of the refined association network to filter and keep only a subset of most important edges using a threshold cutoff value of the edge weight. The degree of a node in the above-described network measures the number of direct associations of a bacterium with other bacteria in the ecosystem. A higher between-ness centrality value of a bacterium node (which is measured by its involvement in connecting other bacteria) could highlight that it is important as a preferred member of the bacterial community. Such nodes correspond to bacterial members showing higher colony forming capability. The key nodes in the refined bacterial association network can hence be identified using the local graph properties and can be studied by the researcher for further insights. This refined bacterial association networks for a disease can then be used for clinical, therapeutic and diagnostic applications for treatment of the disease. For example, methods like NetShift (PMCID: PMC6331612) can be used to identify 'driver' microbes from a case control microbiome study pertaining to a disease using the refined case and control microbiome networks as input. Identified pathogenic driver microbes can be targeted using a therapeutic intervention like probiotics or by alteration of diet or a combination of both in order to cure the disease or improve the health condition.

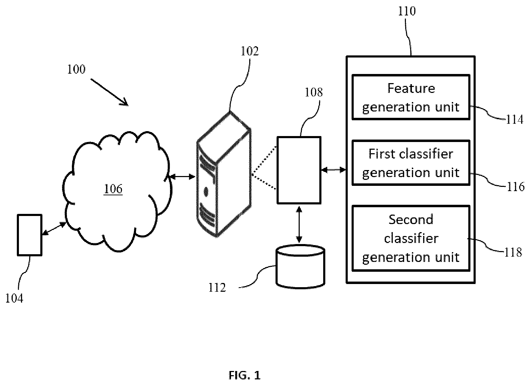

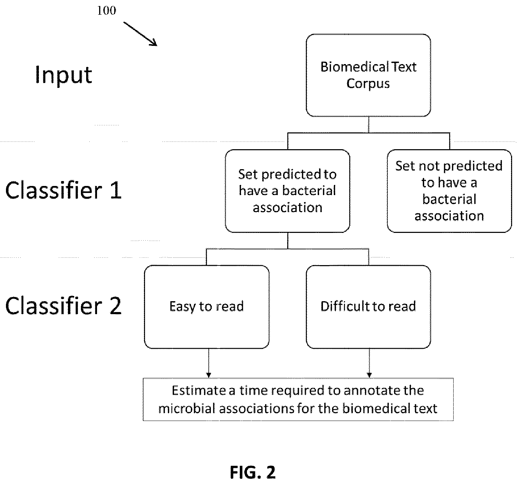

According to an embodiment of the disclosure, FIG. 1 illustrates a network diagram of a system 100 for annotation and classification of biomedical text having bacterial associations. A block diagram of the system 100 for annotation and classification of biomedical text having bacterial associations is shown in FIG. 2.

It may be understood that the system 100 comprises one or more computing devices 102, such as a laptop computer, a desktop computer, a notebook, a workstation, a cloud-based computing environment and the like. It will be understood that the system 100 may be accessed through one or more input/output interfaces 104, collectively referred to as I/O interface 104 or user interface 104. Examples of the I/O interface 104 may include, but are not limited to, a user interface, a portable computer, a personal digital assistant, a handheld device, a smartphone, a tablet computer, a workstation and the like. The I/O interface 104 are communicatively coupled to the system 100 through a network 106.

In an embodiment, the network 106 may be a wireless or a wired network, or a combination thereof. In an example, the network 106 can be implemented as a computer network, as one of the different types of networks, such as virtual private network (VPN), intranet, local area network (LAN), wide area network (WAN), the internet, and such. The network 106 may either be a dedicated network or a shared network, which represents an association of the different types of networks that use a variety of protocols, for example, Hypertext Transfer Protocol (HTTP), Transmission Control Protocol/Internet Protocol (TCP/IP), and Wireless Application Protocol (WAP), to communicate with each other. Further, the network 106 may include a variety of network devices, including routers, bridges, servers, computing devices, storage devices. The network devices within the network 106 may interact with the system 100 through communication links.

The system 100 may be implemented in a workstation, a mainframe computer, a server, and a network server. In an embodiment, the computing device 102 further comprises one or more hardware processors 108, one or more memory 110, hereinafter referred as a memory 110 and a data repository 112, for example, a repository 112. The memory 110 is in communication with the one or more hardware processors 108, wherein the one or more hardware processors 108 are configured to execute programmed instructions stored in the memory 110, to perform various functions as explained in the later part of the disclosure. The repository 112 may store data processed, received, and generated by the system 100.

The system 100 supports various connectivity options such as BLUETOOTH®, USB, ZigBee and other cellular services. The network environment enables connection of various components of the system 100 using any communication link including Internet, WAN, MAN, and so on. In an exemplary embodiment, the system 100 is implemented to operate as a stand-alone device. In another embodiment, the system 100 may be implemented to work as a loosely coupled device to a smart computing environment. The components and functionalities of the system 100 are described further in detail.

According to an embodiment of the disclosure, the memory 110 further comprises a plurality of units. The plurality of units is configured to perform various functions. The plurality of units comprises a feature generation unit 114, a first classifier generation unit 116, and a second classifier generation unit 118. An overview of the classifiers is presented in FIG. 2.

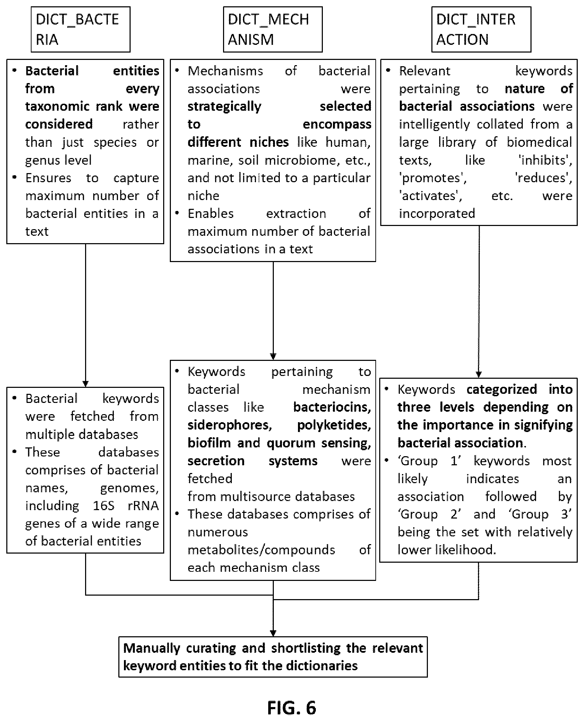

According to an embodiment of the disclosure, the feature generation unit 114 is configured to generate a set of domain features. The system 100 utilizes the set of domain features to represent a biomedical text corpus as multivariate data and utilize the same for classification. The set of domain features is generated as follows: The biomedical text is taken as an input. Basic string pre-processing for the input biomedical text like punctuation and special character removals can be performed followed by sentence tokenization (or splitting a piece of text into individual sentences). A copy of each sentence is also kept without pre-processing for relevant down-stream analysis like entity detection (and their consecutive occurrence detection). Each sentence can then be subjected to a word tokenization (or splitting a piece of text into individual words) followed by classifying each word into their parts of speech like verb, preposition, conjunctions, etc. based on an English or other language dictionary. Following this, a set of domain features is calculated individually for each text chunk (e.g., a biomedical abstract or concise summary of a research paper captured by a set of sentences or a subset/paragraph of a research paper / thesis ideally in the range of approximately 500 words) which forms a part of the biomedical corpus (e.g. having a list of multiple text chunks). These features rely upon three domain dictionaries which has been created specifically for this purpose. The three dictionaries are shown in the flowchart of FIG. 6:

- A first dictionary or "DICT_BACTERIA": A dictionary of bacterial named entities

- A second dictionary or "DICT_MECHANISM": A dictionary of bacterial mechanisms of association

- A third dictionary or "DICT_INTERACTION": A dictionary of terms indicating bacterial associations grouped into three categories (namely group 1, group 2 and group 3) based on their importance

Each sentence corresponding to the biomedical abstract is searched with the DICT_BACT both on a 'word by word' as well as 'bigram' basis. A bigram is a list of two-word sequence of words in a sentence. For example, the sentence "this is an example of bigram" have bigrams like "this is", "is an", "example of" and "of bigram". A temporary bigram dictionary for each sentence of a biomedical abstract is created and each bigram is stored in two steps. While in the first step bigrams are stored intact, the second step modified the first part of the bigram word to include only the first letter followed by a 'dot'. This ensured that bacterial names reported as abbreviated genera as well as with their species names are captured. The occurrences of mechanisms and interaction names are also obtained from each sentence of an input biomedical abstract using the DICT_MECH and DICT_INTR respectively.

In an example, the set of domain features comprises 29 features calculated for a given text chunk. Below is a list of the 29 domain features:

- Feature 1 - Count of total sentences in the biomedical text (TS)
- Feature 2 - Count of the total detected bacterial entities based on DICT_BACT in the biomedical text (TBE)
- Feature 3 - Count of the total detected mechanism entities based on DICT_MECH in the biomedical text (TME)
- Feature 4 - Count of the total detected interaction keywords (TIE) based on DICT_INTR
- Feature 5 - Count of the total detected unique bacterial entities (UB) based on DICT_BACT
- Feature 6 - Count of the total detected unique mechanism entities (UM) based on DICT_MECH
- Feature 7 - Count of the total detected unique interaction keywords (UI) based on DICT_INTR
- Feature 8,9,10 - Total count of keywords from group 1 (TCIG1), group 2 (TCIG2) and group 3 in DICT_INTR found in the biomedical text (TCIG3)

- Feature 11 - Count of the total sentences with at least one detected bacterial entity (TSBE) based on DICT_BACT
- Feature 12 - Count of the total sentences with at least one bacterial entity and at least one mechanism entity (TSBME) based on DICT_BACT and DICT_MECH
- Feature 13 - Total sentences with more than two bacterial entities (TCBE) based on DICT_BACT
- Feature 14 - Size of the largest cluster of bacterial entities (LCBE). A cluster of bacterial entities (or bacterial cluster) is identified in a sentence if more than one bacterial entity is detected consecutively (i.e. one after the other not separated by any other word. They may be however separated by a punctuation or a coordinating conjunction especially "and" as detected by the part of speech tagging). The size of a cluster is calculated as the count of total detected bacterial entities in a cluster. This feature returns the size (or the count value of total bacterial entities) of the highest cluster of bacterial entities based on DICT_BACT present in an input biomedical text.
- Feature 15 - Sum of distance of words between bacterial entities if detected in each sentence (BBDIST) based in DICT_BACT. The distance between two detected bacterial entities in a sentence is calculated as the sum of words (which are not bacterial entities) occurring between them. For clusters of bacterial entities, this feature is calculated only once for all the entities in a cluster. Hence, the BBDIST value is calculated once for a 'bacterial cluster-[separated by few words]-single bacteria', 'bacterial cluster-[separated by few words]- bacterial cluster' and 'single bacteria-[separated by few words]-bacterial cluster'. The distance is calculated for all valid sentences in a text chunk and the final value of BBDIST is the sum of distances for the given text chunk.
- Feature 16-21 - Occurrence of each of the patterns BBM, BMB, MBB, IBB, BIB, BBI in any sentence in the FIXED ORDER of the biomedical text along with the location information of the individual features in the text as indices (i.e. the start and end position of the sentence that contains the feature and the corresponding index positions and name of the bacterial, mechanism and interaction keyword entities in the current text) where: B is the detected BACTERIAL ENTITY NAME based on DICT_BACT, M is the detected MECHANISM of association entity name based on DICT_MECH, I is the detected INTERACTION keyword based on DICT_INTR. The feature returns '1' if the corresponding pattern is present and '0' if it is absent in the biomedical text. Each pattern is treated as a separate feature. A fixed order means that the organization of entities in a sentence belonging to the text chunk follows exactly the same pattern of occurrence one after the other which may or may not be separated by other words and punctuations.
- Feature 22-29 - Occurrence of each of the patterns VB, VBI, VBM, VBMI, VPB, VPBI, VPBM, VPBMI in any sentence of the biomedical text IN ANY ORDER of the biomedical text along with the location information of the individual features in the text as indices (i.e. the start and end position of the sentence that contains the feature and the corresponding index positions and name of the bacterial, mechanism and interaction keyword entities in the current text) where: B is the detected BACTERIAL ENTITY NAME based on DICT_BACT, M is the detected MECHANISM of association entity name based on DICT_MECH, I is the detected INTERACTION keyword based on DICT_INTR, V is a VERB as detected by the parts of speech tagging, P is a PREPOSITION as detected by the parts of speech tagging. The feature returns '1' if the corresponding pattern is present and '0' if it is absent in the biomedical text. Each pattern is treated as a separate feature. Any order means that the organization of entities in a sentence belonging to the text chunk may not exactly follow the same pattern of occurrence one after the other.

In summary, the set of domain features calculated from biomedical corpus consists of a plurality of compositional features and a plurality of context aware features. The plurality of compositional features include ones like total and unique entity counts, sentence specific entity counts and entity presence in combination with various parts of speeches (e.g., Features 1 to 13). On the other hand, the plurality of context aware features include count of one or more entity patterns with or without in combination with parts of speeches in a given order (fixed or any order) in one or more sentences, sum of word distance between bacterial entities and size of largest clusters of consecutively occurring bacterial entities (e.g., Features 14 to 29).

According to an embodiment of the disclosure, the system 100 can also be configured to provide clinical, therapeutic and diagnostic applications for treatment of a disease. In many diseases having a known bacterial basis, it is often essential to decipher the bacterial community structure in order to gather improved understanding on the same. Insights on bacterial community structure is best obtained from bacterial association networks where the participating bacterial taxon serves as nodes and their relationship serves as the edge for the network graph. One of the most common ways to obtain such bacterial association networks is by utilizing experimental microbiome data obtained from one or more metagenomic (whole genome sequencing), amplicon (e.g. 16s rRNA gene) sequencing or microscopy-based study. In such studies, the experiment aims to collect genetic material of all the microbes directly from the environmental samples and analyzing the same further computationally.

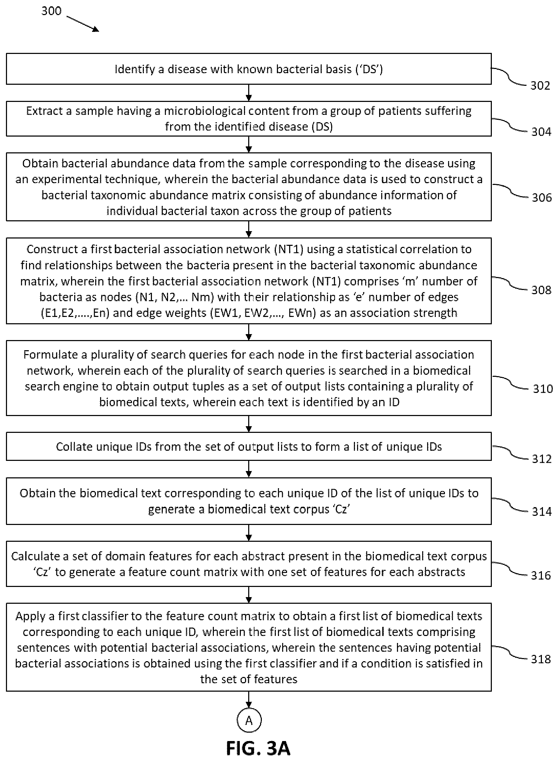

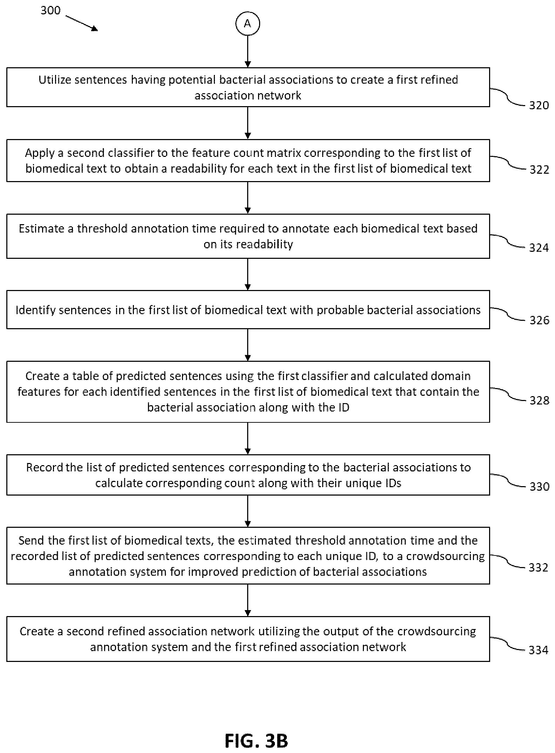

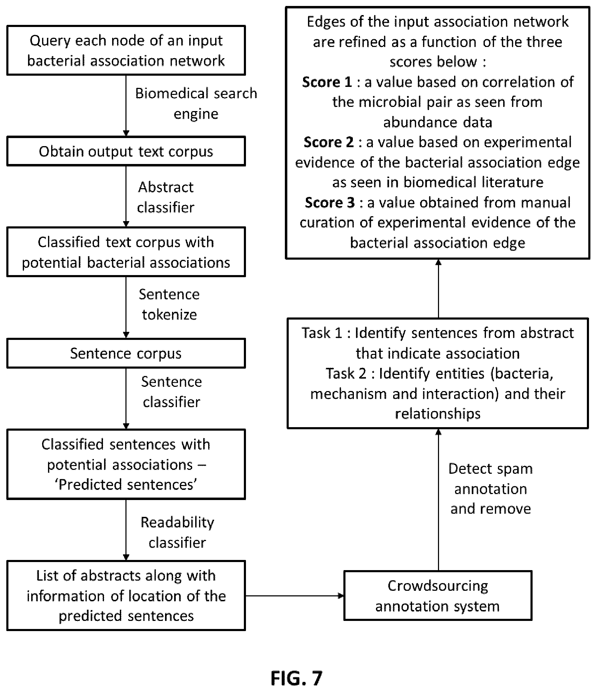

In operation, a flow diagram of a method 300 for annotation and classification of biomedical text having bacterial associations and further utilizing for the disease is shown in FIG. 3A and 3B. The method 300 depicted in the flow chart may be executed by a system, for example, the system, 100 of FIG. 1. In an example embodiment, the system 100 may be embodied in a computing device. FIG. 7 shows a simplistic flow diagram of the method 300 for annotation of the biomedical text.

Operations of the flowchart, and combinations of operation in the flowchart, may be implemented by various means, such as hardware, firmware, processor, circuitry and/or other device associated with execution of software including one or more computer program instructions. For example, one or more of the procedures described in various embodiments may be embodied by computer program instructions. **In** an example embodiment, the computer program instructions, which embody the procedures, described in various embodiments may be stored by at least one memory device of a system and executed by at least one processor in the system. Any such computer program instructions may be loaded onto a computer or other programmable system (for example, hardware) to produce a machine, such that the resulting computer or other programmable system embody means for implementing the operations specified in the flowchart. It will be noted herein that the operations of the method 300 are described with help of system 100. However, the operations of the method 300 can be described and/or practiced by using any other system.

Initially at step 302 of the method 300, a disease is identified with a known or reported bacterial basis. Let the disease be named as 'DS'. At step 304, a sample having a microbiological content is extracted from a group of patients suffering from the identified disease for investigating a disease. The environmental sample can be taken from fecal matter, saliva, swabs, etc. (or any sample having a microbiological content) from the subjects under consideration. The extracted genetic matter (like the DNA) from the environmental samples is then sequenced and the sequences are computationally analyzed to identify the bacterial taxa present by mapping the same to a database of microorganisms of various taxonomic hierarchy.

At step 306 of the method 300, bacterial abundance data is obtained from the sample corresponding to the disease using an experimental technique, wherein the bacterial abundance data is used to construct a bacterial taxonomic abundance matrix consisting of abundance information of individual bacterial taxon across the group of patients. The frequency of mapping of signature genetic elements (e.g. 16s rRNA marker genes) can be used to estimate the abundances of the constituent microbes in the environmental sample. This constitutes the bacterial taxonomic abundance matrix for a given microbiome study consisting of abundance information of individual bacterial taxon across multiple subjects (or how frequently each bacterium is present for each subject under analysis). One or more of such matrices obtained from multiple metagenomic projects can be combined given they are performed and analyzed under similar experimental setup, parameters and conditions.

A data normalization step is desirable in the next step to remove various sampling and experimental biases. The most common technique of normalization being a total sum scaling or a percentage normalization. However, advanced techniques including rarefaction based or centered log-ratio based transformation may also be used to normalize the abundance matrices. The bacterial taxonomic abundance matrix can then be used to identify relationship patterns between the bacteria (in the matrix) using proxy measures like significant correlations calculated from the matrix. A significant positive correlation between a pair of bacteria from the matrix can indicate a mutual association pattern while a significant negative correlation can serve as an indicator of a mutual inhibitory relationship between them. While the significance of an association can be ascertained by a statistical test using a probability value, the association itself can also be calculated by other computational methods. It should be noted that other methods for indirect correlation and mutual information extraction can also be used for this step. Once the 'all versus all' pairwise bacterial associations (or in other words all possible unique pairwise combinations between the available bacteria) are calculated from the bacterial taxonomic abundance matrix, only the significant associations above or below a certain threshold value (which can be the correlation or the probability value of the association) are identified and an edge is connected between that pair of **bacteria** having a significant association (for example correlations having a probability value < 0.05). Upon completion of the task for all the bacteria in the matrix, a bacterial association network is generated.

The bacterial association generated in the above step (from a given bacterial taxonomic abundance matrix) can serve as a starting for analysis of the bacterial community structure corresponding to a study (e.g., the diseases having a known bacterial basis). However, not all correlations inferred from an abundance data are true indicators of an association. Experimental evidence of an association between a given set of bacteria is hence required to ascertain the bacterial relationships indicated by the bacterial association network. The set of steps presented in this disclosure can help achieve this task by obtaining evidence information pertaining to a given set of bacterial associations from biomedical literature of scientific texts reporting experimental findings (e.g. databases like PubMed central).

At step 308, a bacterial association network is constructed using significant statistical correlation or other methods to find relationships between the bacteria present in the bacterial taxonomic abundance data matrix. Consider that this network (NT1) has 'm' number of bacteria as nodes (N1, N2....Nm) with their relationship as 'e' number of edges (E1,E2,...., En) and edge weights (EW1, EW2,..., EWn) as an association strength (e.g. correlation along with its probability values). The association strength is identified using score 1 which is obtained using the value of correlation. The score 1 can be range scaled to lie between 0 and 1 and identified with a probability value (ps1) of the correlation calculated using a test statistic (e.g., t distribution).

At step 310 of the method 300, a plurality of search queries is formulated for each node in the first bacterial

**EP 4 125 091 B1**

association network (NT1), wherein each of the plurality of search queries is searched in a biomedical search engine to obtain output tuples as a set of output lists containing a plurality of biomedical texts, wherein each text is identified by an ID. Search queries are formulated for each node in the network using a biomedical search engine (like PubMed). Each search query is designed to fetch the plurality of biomedical text that contain the name of the search bacterial taxa node and obtain the output tuples (e.g., PubMed IDs with biomedical abstracts) as 'z' output lists. At step 312, unique IDs (e.g., PubMed IDs) is collated from the set of output lists to form a list of unique IDs. In the next step 314, the biomedical text (e.g., Publication abstracts) is obtained corresponding to each unique ID of the list of unique IDs to generate a biomedical text corpus 'Cz'.

At step 316 of the method 300, the set of domain features is calculated for each of the abstracts present in the biomedical text corpus 'Cz' to generate a feature count matrix with one set of features for every abstract. Further, at step 318, a first classifier (or abstract classifier trained on the abstract corpus) is applied to the feature count matrix to obtain a first list of biomedical texts corresponding to each unique ID, wherein the first list of biomedical texts comprising sentences with potential bacterial associations, wherein the sentences having potential bacterial associations is obtained using the first classifier (or sentence classifier trained on the sentence corpus) and if a condition is satisfied in the set of features. Further at step 320, sentences having potential bacterial associations are utilized to create a first refined association network. It must be noted that, the first classifier can be trained to detect both abstracts as well as sentences containing bacterial associations.

At step 322 of the method 300, a second classifier (or readability classifier) is applied to the feature count matrix corresponding to the first list of biomedical text to obtain a readability for each text in the first list of biomedical text. A feature count matrix is a two-dimensional matrix composed of the abundance of each feature across each unique ID (e.g., PubMed IDs) of the biomedical corpus. Further at step 324, a threshold annotation time required to annotate each biomedical text is estimated based on its readability;

In the next step 326 of the method 300, sentences in the first list of biomedical text are identified (by a method like sentence tokenization) with probable bacterial associations. At step 328 a table of predicted sentences is created using the first classifier (or sentence classifier trained on the sentence corpus) and calculated domain features for each identified sentences in the first list of biomedical text that contain the bacterial association along with the ID. Additional information for each sentence in the table including the source ID (biomedical abstract that contains the sentence), output of features 16 to 21, information about the presence and location of the bacterial mechanism and the interaction entities from the identified sentences are also included in the table. At step 330, the list of predicted sentences corresponding to the bacterial associations is recorded to calculate corresponding count along with their unique IDs.

At step 332 of the method 300, the first list of biomedical texts, the estimated threshold annotation time and the recorded list of predicted sentences corresponding to each unique ID is sent to a crowdsourcing annotation system for improved prediction of bacterial associations. And finally, at step 334, a second refined association network is created utilizing the output of crowdsourcing annotation system and the first refined association network.

According to an embodiment of the disclosure, the method 300 further comprises calculating a refined bacterial association network. Initially, the sentences with bacterial entities, interactions entities and mechanism entities are identified for the list of biomedical texts, wherein the bacterial entities mentioned in the sentences are connected by an edge. In the next step, a total occurrence of the edge is counted across the sentences in the lists and a normalized edge weight is assigned. In other words, the edge weight (or score of a given edge) is identified using score 2 which is calculated as a ratio of the count of text chunk in the text corpus 'Cz' where a successful presence of the association is predicted using classifier 1 to the count of the text chunk in the text corpus 'Cz' containing both the bacterial nodes constituting the edge. The score 2 can be range scaled to lie between 0 and 1 and a probability (ps2) value is calculated for the bacterial association pair using a statistical test like Fisher's Exact Test.

Further, a second bacterial association network (NT2) is generated with 'o' number of nodes (N1, N2,...,No) and 'p' number of edges (E1, E2,...., Ep) with normalized edge weights (EW1, EW2,...., EWp) as identified using score 2. Finally, one or more common edges are found present in the first bacterial association network NT1 and the second bacterial association network NT2 to calculate a refined bacterial association network NT3 with intersection edges having 'q' number of nodes (N1, N2,.....,Nq) and 'r' number of edges (E1,E2,.....,Er) with edge weight (EW1, EW2,.., EWr) as a function of the edge weights of the NT1 and NT2 identified using score 1 and score 2. This allows to obtain a first refined bacterial association network for improved insights.

According to an embodiment of the disclosure, the crowdsourcing annotation system consists of the following tasks assigned to an annotator:

Task 1: Identify the sentences in a given text chunk (e.g., biomedical abstract) that indicates a probable bacterial association. The sentence can be selected by a text highlighting feature which in turn can be used to capture its actual start and end index in the current text chunk. The annotator can also manually copy and paste the relevant sentences in a provided text box. The identified sentences are saved in a list 'Annotated sentences'. The set of sentences in the table of 'Predicted-sentences' for each text chunk and the associated information including entity information can be optionally highlighted in the displayed user interface to lower annotator load.

Task 2: Identify the possible bacteria, mechanism and interaction entities in a given text chunk and assign a relationship between the observed bacteria names visible in the text chunk by human comprehension. Option to show/hide the automatically detected annotations using list of 'Predicted-sentences' and table can be used to lower annotator load. The relationship can be identified by selecting the bacterial names, relationship and mechanism from a text dropdown or similar GUI based menu populated automatically in the crowdsourcing annotation system. The annotator can also manually list the observations in a provided text box indicating the exact bacteria entity names, their mechanism and interaction as visible in the text chunk.

In addition, the crowdsourcing annotation system can also record the time taken by an annotator (T-actual) to successfully complete an annotation as well as track annotator attentiveness. This can be achieved in several ways but not limited to tracking the mouse cursor, touch delays, eye tracking, active time of the user interface page, etc. Each text chunk is also annotated by two other independent annotators. The reliability and accuracy of an annotation is then calculated using the following ways:

- The actual annotation time (T-actual) for an annotator is compared with the threshold annotation time range (T-threshold) based on the readability predicted for the text chunk. If the actual annotation time is greater than the maximum or lower than the minimum threshold annotation time range, the annotation is sent for a manual verification as it may be a spam. Readings obtained from annotator attentiveness can also be coupled with the annotation time to detect spam annotations. If the manual verification ascertains an association, the annotation is confirmed.
- Each sentence is expected to be annotated by at least three independent annotators. The sentences common between at least two of the three independent verified annotators (i.e. non spam annotation) for each text chunk (identified with a unique ID e.g. the PubMed ID or the search ID) are identified and saved in a list 'Consensus-annotated'. Alternate scoring schemes (as described in a later part of the specification) can also be used to assign weightage to annotators and subsequently use the same for scoring the reliability of an annotation.

The list of sentences corresponding to the 'Consensus-annotated' for each text corpus is processed to further refine the bacterial association network by modifying the edge weights and create the second refined association network such that its edge weight is a function of score 1, score 2 and score 3, where,

- score 1 = A correlation value of abundance count calculated between the two bacteria (as nodes) forming the bacterial association edge from a microbiome experiment (e.g., Pearson correlation coefficient, Spearman's rank correlation coefficient, etc.)
- score 2 = A score of experimental evidence of the bacterial association edge as seen in biomedical literature (e.g., a normalized count of observations reporting the bacterial association with respect to a text corpus)
- score 3 = A score obtained from manual curation of experimental evidence of the bacterial association edge (e.g., a normalized count of annotations for the evidence annotated for the bacterial association edge by a set of annotators)

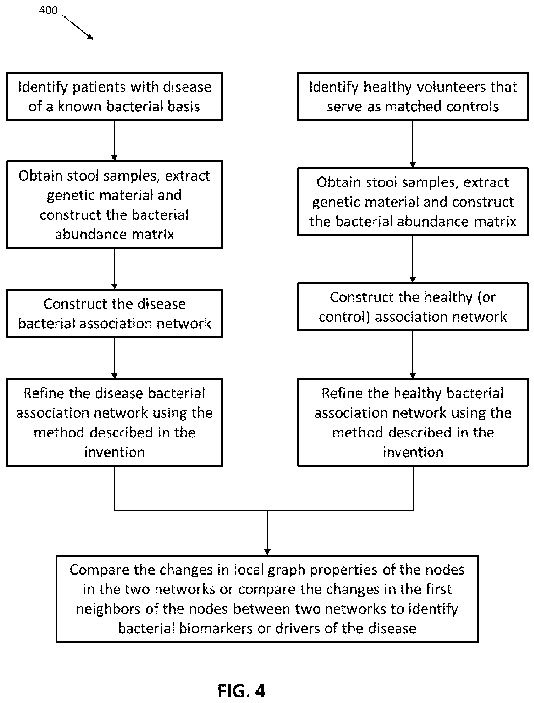

According to an embodiment of the disclosure, the refined bacterial association networks for the disease can also be used for clinical, therapeutic and diagnostic applications for treatment of the disease as explained above. For example, microbial/bacteria biomarkers and drivers for a disease can be identified for by comparing the refined disease bacterial association network with a similarly refined healthy (control) bacterial association network obtained from matched heathy control sample data as shown in flowchart 400 of FIG. 4. As shown in the figure, the refined bacterial association network is prepared for the disease sample and the healthy sample. The changes in local graph properties of the nodes in the two networks are then compared to identify bacterial biomarkers or drivers of the disease.

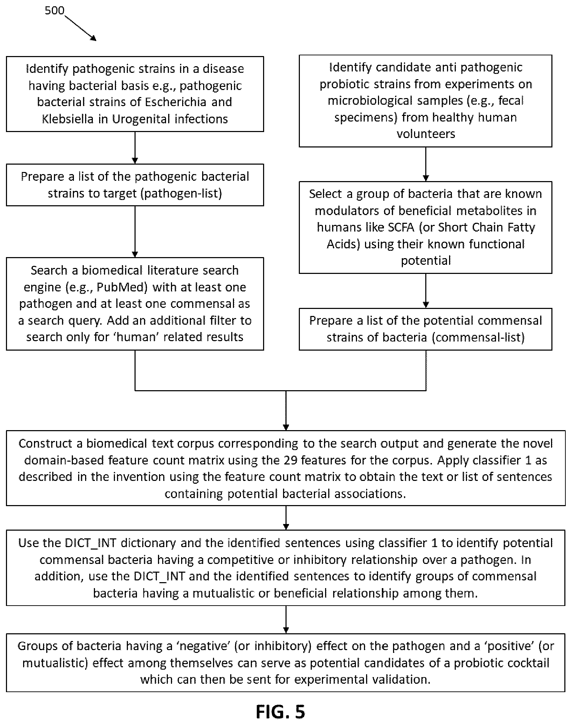

According to an embodiment of the disclosure, the bacterial association network can also be used to augment the understanding of the functional relationships between the bacterial groups. Advanced probiotic cocktails can then be designed using this refined bacterial association networks as reference which minimizes all non-naturally feasible bacterial associations as shown in flowchart 500 of FIG. 5. In such a use case, first a list of pathogenic drivers or microbial strains pertaining to a disease (known to have a bacterial basis) are identified and prepared, e.g., pathogenic bacterial strains of *Escherichia* and *Klebsiella* in urogenital infections. On the other hand, a list of candidate anti pathogenic probiotic strains from experiments on microbiological samples (e.g., fecal specimens) from healthy human volunteers are identified using a probiotic strains database. This probiotic strain database can be created using one or more clinically approved and recommended list of probiotics. An alternate way is by selecting a group of bacteria that are known modulators of beneficial metabolites in humans like SCFA (or Short Chain Fatty Acids) using their known functional potential available from metabolic pathway and protein domain information. Next, a biomedical literature search engine is queried with at least one pathogen and at least one commensal from the list as a search query with an additional filter to search only for human related results. This tries to ensure that the candidate bacterial strains of the probiotic cocktail are mostly of reported human origin.

Once the search is complete, the biomedical text corpus is created corresponding to the search output and the domain-based feature count matrix is generated using the set of domain features described in the disclosure for the corpus. Further, the classifier 1 is applied as described in the disclosure using the feature count matrix to obtain the text or list of sentences containing potential bacterial associations. Next, the DICT_INT dictionary and the identified sentences using classifier 1 is used to identify potential commensal bacteria having a competitive or inhibitory (negative) relationship over a pathogen. In addition, the DICT_INT dictionary and the identified sentences are used to identify groups of commensal bacteria having a mutualistic or beneficial (positive) relationship among them. The identified groups of bacteria having a negative (or inhibitory) effect on the pathogen and a positive (or mutualistic) effect among themselves serve as potential candidates of a probiotic cocktail which can then be sent for experimental validation. Identification of mutualistic and inhibitory relationships between a pair of bacteria in a given classified sentence can be done either by manual curation or by application of machine learning methods for relationship prediction. It is pertinent to note that although the example case study demonstrates the applicability of the methodology using a specific disease, the method presented in this invention can be well extended to study various other bacterial ecosystems in diverse ecological regimes. Refined bacterial association networks generated from multiple studies can be combined to create a knowledge graph of bacterial associations along with other information like bacteria-disease, bacteria-food, bacteria-drug, bacteria-host genetic/epigenetic factors, bacteria-functions, bacteria-active substances, bacteria-virus, etc. Such knowledge graphs can be used by researchers and clinicians to design personalized recommendation systems pertaining to diet, drug, probiotics and prebiotics. Such knowledge graphs can also be used for discovering and designing novel drug candidates utilizing information of bacterial association and metabolites secreted by them using the dictionaries and classifiers presented in this invention. For example, one can identify structurally similar molecules secreted by one or more bacteria or bacterial community for a drug known to alleviate a disorder or improve health condition in a disease. Such identification can help to engineer and reuse the bacterial community as a natural alternative for the drug in order to minimize adverse effects.

According to an embodiment of the disclosure the steps for the creation of data set for classifier training and validation, classifier 1 and classifier 2 is provided below. Relevant biomedical abstracts were downloaded from PubMed using keywords corresponding to bacterial associations and mechanisms. Unlike other relationships, bacterial associations require a special categorical evaluation. Hence, we introduce four categories which are required for evaluation of a classifier build for classifying bacterial associations. The downloaded corpus was then curated computationally for validation of 'association classification' (Classifier 1) to create these four categories namely, CATEGORY 1, CATEGORY 2, CATEGORY 3a and CATEGORY 3b (consisting of 300 abstracts belonging to each category with a total of 1200 abstracts):

- CATEGORY 1 (CAT1): Abstracts having no BACTERIA and MECHANISM names. This category primarily contains a set of abstracts which are irrelevant from the point of identification of bacterial associations.
- CATEGORY 2 (CAT2): Abstracts having only BACTERIA names but no relevant MECHANISM or INTERACTION names. This category primarily consists of abstracts that mention about the presence of bacteria in an environment, habitat, experimental setup but provide no indications about the inter bacterial associations.
- CATEGORY 3 (CAT3): Abstracts having BACTERIA, MECHANISM and INTERACTION names. The abstracts corresponding to CAT1 and CAT2 were then manually examined to include 300 abstracts from each. A thorough manual curation by a group of domain based annotators (working in the field of microbiome) was then performed on the abstracts under CAT3 to identify two subsets of size 300 each namely:
- CATEGORY 3a (CAT3a): Abstracts consisting of one or more identified inter bacterial association/interaction post manual curation with or without a reported mechanism. These abstracts have BACTERIA, INTERACTION and optional MECHANISM names. This category primarily constitutes a set of abstracts that serve as the best source to extract information pertaining to inter bacterial associations.
- CATEGORY 3b (CAT3b): Abstracts consisting of no identified inter bacterial association/interactions although having BACTERIA, INTERACTION and optional MECHANISM names. This category contains abstracts which although look like ones having reported bacterial association based on the joint occurrence of different entity names. However, upon closer examination they in reality do not have any reported bacteria-bacteria association/interaction.

In essence, the CAT3a represents a 'TRUE' or 'POSITIVE' class while CAT1, CAT2 and CAT3b represents three types of 'FALSE' or 'NEGATIVE' classes encountered normally in biomedical text mining especially with respect to bacterial associations. 'TRUE' or 'POSITIVE' in this case refers to a text reporting one or more identifiable bacterial association. It is pertinent to note that, among the above classes, the most difficult task is to distinguish between CAT3a ('TRUE' or 'POSITIVE' class) and CAT3b ('FALSE' or 'NEGATIVE'). Following is an example of a sentence belonging to abstracts corresponding to CAT2, CAT3a and CAT3b. "Although the presence of *Treponema spp., Fusobacterium necrophorum and Porphyromonas levii* was confirmed by fluorescence in situ hybridization (FISH), the results for

*Mycoplasma sp.* were inconclusive" is an example of a sentence belonging CAT2. **"Leucocin C,** produced by *Leuconostoc carnosum* 4010, is a class IIa **bacteriocin** used to **inhibit** the growth of *Listeria monocytogenes"* is an example of a sentence belonging to CAT3A. "An extract from *Sargassum horridum* was the only one that reversed the resistance to **antibiotics against** both *Staphylococcus aureus* and *Streptococcus pyogenes"* is an example of a sentence belonging to CAT3b. In the above examples bacterial entities like *Treponema spp., Fusobacterium necrophorum, Porphyromonas levii, Mycoplasma sp. , Leuconostoc carnosum, Listeria monocytogenes, Staphylococcus aureus, Streptococcus pyogenes* can be identified using DICT_BACT. Mechanism entities like **bacteriocin** and **antibiotics** can be identified using DICT_MECH. Interaction entities like **inhibit** and **against** can be identified using DICT_INT.

A list of biomedical text corpus was also manually scored for their readability and divided into two classes 'Easy' (count = 100) and 'Difficult' (count = 100) which was used for Classifier 2.

**Creation of Classifier 1 for 'association classification'**

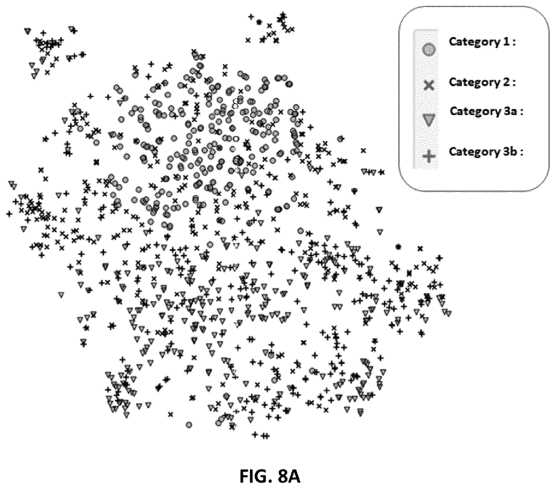

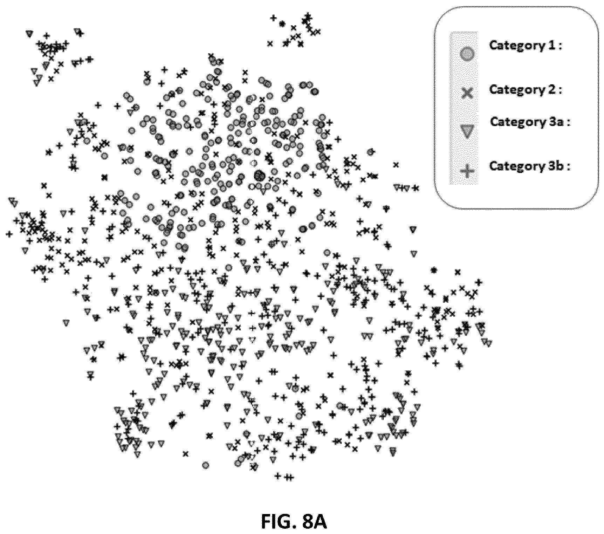

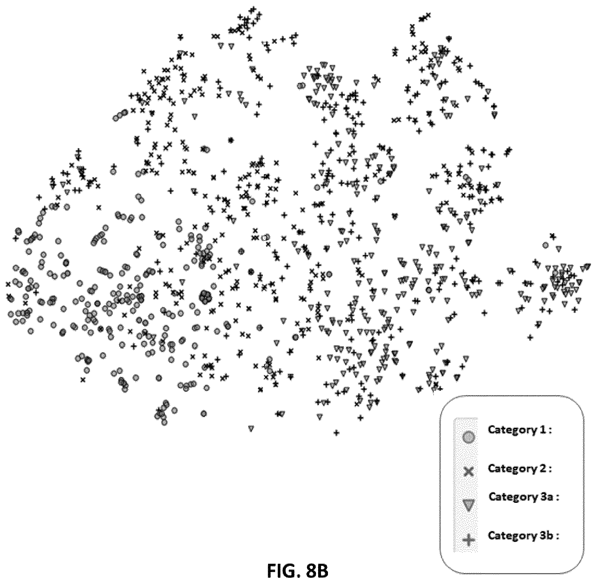

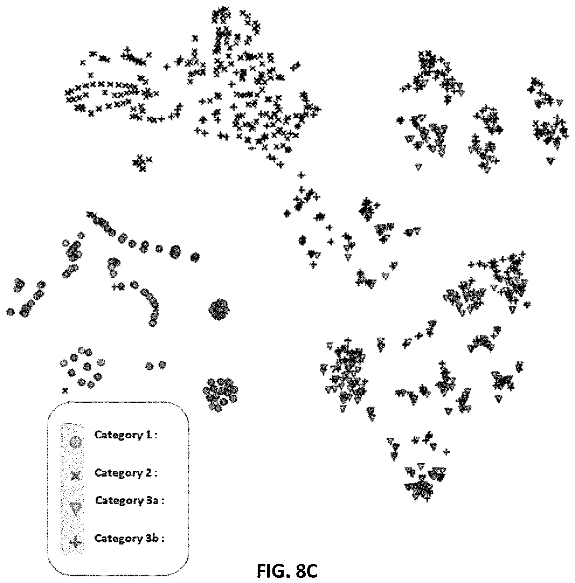

All the biomedical abstracts corresponding to the four categories of the main corpus were preprocessed and the entities namely bacteria, mechanism and interaction were identified using the corresponding dictionaries. For bacterial entity, each sentence corresponding to a biomedical abstract was searched with the DICT_BACT both on a 'word by word' as well as 'bigram' basis. A temporary bigram dictionary for each sentence of a biomedical abstract was created and each bigram was stored in two steps. While in the first step, bigrams were stored intact, the second step modified the first part of the bigram word to include only the first letter followed by a 'dot'. This ensured that bacterial names reported as abbreviated genera as well as with their species names are captured. An alternate way to capture bacterial entity names is by adding all variations of naming a microbial taxon in the DICT_BACT itself. Following this, the 'domain based' feature extraction utilizing feature 1 to 29 was carried out and a multivariate feature matrix (1200 abstracts versus 29 features) of the whole biomedical text corpus was generated. In order to visualize the inter data point (text files having biomedical abstracts) similarities in a multivariate space, a tSNE plot was generated using Orange tool using the default parameters for the three types of features namely, Bag of words or BOW as shown in FIG. 8A, TF-IDF shown in FIG. 8B and domain features as shown in FIG. 8C. tSNE is a method for dimensionality reduction and visualization of high dimensional data, minimizes the divergence between pairs of data points in the high dimensional space and low dimension space, the points closer in high dimensional space remained close in the low dimensional space. The tSNE plots were generated to visualize the overall distribution of the four classes in multivariate space and obtain an initial idea of the clustering efficiency of the different types of features. A comparative visual inspection shows that the 'set of domain features' are able to better distinguish between the different categories as compared to the generic features namely BOW and TF-IDF. This was evident from the observation of datapoints belonging to same clusters having closer proximity and datapoints belonging to different clusters having a larger proximity in the two-dimensional ordination space as generated by tSNE. In addition to visual inspection, any cluster quality index known in art like silhouette index can be used for computational evaluation of cluster quality.

A multiclass (Category 1 vs Category 2 vs Category 3a vs Category 3b) classification **('classifier 1')** was performed using four algorithms namely Naive Bayes, Logistic Regression, SVM and Random Forests. Optimum hyperparameters for SVM and Random Forest classifiers are obtained using Grid Searching. For Bag-of-words and TF-iDF models, top 100 features were considered with up to 4-grams. The algorithms were trained on 90% samples and tested on 10% chosen randomly. Optimum parameter estimation is done by 5-fold cross validated grid searching on a predefined parameter grid. During each parameter search operation, the training samples are split into 5 segments, wherein 4 are used for training and 1 for validation. However, as this classification takes a random set of training and test data from the main corpus, a 100-step bootstrap validation was performed for all the classifiers in order to avoid biases in the result. For every bootstrap step, a random 90% of samples were chosen as a training set and a random 10% as a test set. The mean values of the bootstrapped output are tabulated below in TABLE 1.

TABLE 1: Comparative performance of classifier 1 to distinguish between all the four categories shows that the set of domain features outperform other generic feature based classifiers. BOW denotes Bag of Words features, TFIDF corresponds to TFIDF features.

| BOW | Precision | Recall | F1 Score |
|---|---|---|---|
| Naive Bayes | 0.704466 | 0.710417 | 0.689975 |
| Logistic Regression | 0.698316 | 0.7 | 0.690899 |
| SVM | 0.723233 | 0.723333 | 0.718293 |
| Random Forest | 0.759754 | 0.760667 | 0.753178 |
| | | | |

(continued)

| TFIDF | Precision | Recall | F1 Score |
|---|---|---|---|
| Naive Bayes | 0.718571 | 0.720917 | 0.703679 |
| Logistic Regression | 0.737998 | 0.740167 | 0.733554 |
| SVM | 0.756018 | 0.755583 | 0.751546 |
| Random Forest | 0.762485 | 0.763417 | 0.75396 |
| | | | |
| **Domain features** | Precision | Recall | F1 Score |
| Naive Bayes | 0.859626 | 0.815583 | 0.795638 |
| Logistic Regression | 0.876156 | 0.875083 | 0.872834 |
| SVM | 0.883042 | 0.881167 | 0.880157 |
| Random Forest | 0.898127 | 0.896 | 0.895563 |

The results indicate that the set of domain features outperforms other methods using generic features like 'Bag of words' and 'TF-IDF'. All the measures including precision, recall and F1 score were best in all the four classifiers using the domain features namely Naive Bayes, Logistic regression, Support Vector Machine (SVM) and Random forest classifier. Further, in order to evaluate the ability of the 'set of domain features' to differentiate between the two classes '3a' and '3b', the same classifier was tested with similar parameters using only the CATEGORY 3a and CATEGORY 3b as input. The results (as shown in TABLE 2). Similar to the capability to distinguish between the four classes, the results show that the 'set of domain features' also outperform in distinguish between the two sub-categories as well in all classifiers except Naive Bayes.

Table 2: Comparative performance of classifier 1 to distinguish between category 3a and category 3b shows that the domain based features outperform other generic feature based classifiers. BOW denotes Bag of Words features, TFIDF corresponds to TDIDF features.

| BOW | Precision | Recall | F1 Score |
|---|---|---|---|
| Naive Bayes | 0.708811 | 0.6885 | 0.680623 |
| Logistic Regression | 0.746506 | 0.739 | 0.738116 |
| SVM | 0.751461 | 0.744833 | 0.744379 |
| Random Forest | 0.789838 | 0.783667 | 0.783215 |
| | | | |
| **TFIDF** | Precision | Recall | F1 Score |
| Naive Bayes | 0.739543 | 0.719 | 0.715645 |
| Logistic Regression | 0.757367 | 0.75 | 0.750031 |
| SVM | 0.775759 | 0.765333 | 0.765017 |
| Random Forest | 0.786713 | 0.7785 | 0.778621 |
| | | | |
| **DOMAIN features** | Precision | Recall | F1 Score |
| Naive Bayes | 0.761332 | 0.664333 | 0.630482 |
| Logistic Regression | 0.818864 | 0.813333 | 0.813407 |
| SVM | 0.810776 | 0.805333 | 0.805399 |
| Random Forest | 0.837367 | 0.831 | 0.831015 |

In the next step, a sentence corpus is created consisting of a set of 405 sentences identified by manual curation to contain reported microbial associations marked as TRUE and another set of 405 sentences manually curated to identify the ones having no bacterial associations marked as FALSE (although having the bacteria/mechanism/interaction entity names present in them). These sentences were obtained using manual curation from the 1200 abstracts (text corpus) belonging to the above described four categories (CAT1, CAT2, CAT3a and CAT3b). A classification was performed using four algorithms namely Naive Bayes, Logistic Regression, SVM and Random Forests. Optimum hyper-parameters for SVM and Random Forest classifiers are obtained using Grid Searching. For Bag-of-words and TF-iDF models, top 100 features were considered with up to 4-grams. The algorithms were trained on 90% samples and tested on 10% chosen randomly. Optimum parameter estimation is done by 5-fold cross validated grid searching on a predefined parameter grid. During each parameter search operation, the training samples are split into 5 segments, wherein 4 are used for training and 1 for validation. However, as this classification takes a random set of training and test data from the main corpus, a 100-step bootstrap validation was performed for all the classifiers in order to avoid biases in the result. For every bootstrap step, a random 90% of samples were chosen as a training set and a random 10% as a test set. The mean values of the bootstrapped output are tabulated below in TABLE 3. Similar to the capability to distinguish between the four classes, the results show that the 'set of domain features' also outperform to distinguish bacterial associations at a sentence level.

TABLE 3: Comparative performance of classifier 1 to distinguish between 'TRUE' and 'FALSE' categories belonging to the sentence corpus. The results show that the set of domain features outperform other generic feature based classifiers. BOW denotes Bag of Words features, TFIDF corresponds to TDIDF features.

| BOW | Precision | Recall | F1 Score |
|---|---|---|---|
| Naive Bayes | 0.682525 | 0.674815 | 0.673336 |
| Logistic Regression | 0.741613 | 0.732469 | 0.731382 |
| SVM | 0.734451 | 0.727654 | 0.727127 |
| Random Forest | 0.720243 | 0.714815 | 0.714514 |
| | | | |
| TFIDF | Precision | Recall | F1 Score |
| Naive Bayes | 0.696752 | 0.691852 | 0.691408 |
| Logistic Regression | 0.740223 | 0.735309 | 0.735188 |
| SVM | 0.738547 | 0.733086 | 0.732836 |
| Random Forest | 0.754063 | 0.748148 | 0.747854 |
| | | | |
| DOMAIN features | Precision | Recall | F1 Score |
| Naive Bayes | 0.745941 | 0.739136 | 0.738872 |
| Logistic Regression | 0.792868 | 0.785802 | 0.785615 |
| SVM | 0.812271 | 0.804815 | 0.804421 |
| Random Forest | 0.798489 | 0.792469 | 0.792491 |

As evident from the results presented in TABLE 1, 2 and 3, the classifiers trained with the 'set of domain features' are able to better distinguish both abstracts (a text corpus with multiple sentences) as well as individual sentences having bacteria associations. In addition to better Precision and Recall, the classifiers also show an overall high F1 score in all the cases. In addition to that, it is pertinent to note that, the presented 'set of domain features' being less in number (count = 29), they are expected to make the process of computation less complex both in space and time complexity.

To estimate the individual feature's contribution to the classifiers, a feature importance score was computed for each feature pertaining to each of the classifications as demonstrated by the classifier 1 in TABLE 1, 2 and 3. The feature importance was calculated for the Random Forest classifier using the Gini score or index measure. The Gini index is given by:

$$Gini = 1 - \sum_{i=1}^{n}(p_i)^2 \ldots\ldots\ldots\ldots\ldots (1)$$

where $p_i$ is the probability of an object being classified to a particular class. Gini score measures the probability of a particular variable being wrongly classified. A feature is given a higher importance, if its elimination from the feature set causes the Gini coefficient of the data to increase. Importance scores (as shown in TABLE **4)** are therefore calculated as the normalized total reduction in Gini due the absence of that feature. Higher the importance score, more importance does a feature hold for the corresponding classification. In simple words, a feature is deemed important, if its presence increases the information about the sample. For example, if the presence of interaction keywords is considered as a feature, then it is safe to conclude that the presence of this feature in any sentence should increase the chances of a reported interaction in that sentence. In the decision tree, the aim of every split is to decrease the Gini score of the subsets. A branch stops splitting further if its Gini score=0, or in other words, it has items from a single class only. A feature's importance can thus be estimated by the fraction of Gini score lost when that feature is eliminated from the tree. In a trained random forest, feature importance is the reduction in Gini score due to the absence of that feature, averaged over all the trees in the forest. The final feature importance scores were calculated using the average score of each output across 100 bootstrap iterations.

| (A) CAT1+CAT2+CAT3a+CAT3b | | (B) CAT3a+CAT3b | | (C) Sentence (TRUE+FALSE) | |
|---|---|---|---|---|---|
| Feature | Importance | Feature | Importance | Feature | Importance |
| TSBE | 0.104773 | TIE | 0.102706 | BBdist | 0.202476 |
| TME | 0.100837 | TCIG1 | 0.101086 | BIB | 0.103981 |
| UB | 0.082905 | UI | 0.091874 | TCIG1 | 0.087191 |
| TBE | 0.078138 | BIB | 0.088318 | TIE | 0.077705 |
| UM | 0.068729 | BBdist | 0.064686 | UB | 0.072518 |
| TIE | 0.068262 | TME | 0.058636 | UI | 0.061781 |
| VPB | 0.056096 | TSBME | 0.051301 | LCBE | 0.046375 |
| UI | 0.055394 | TBE | 0.042992 | TBE | 0.043331 |
| TCIG1 | 0.05367 | TSBE | 0.042781 | TCIG3 | 0.035191 |
| VB | 0.044077 | UB | 0.042238 | TME | 0.030088 |
| BBdist | 0.031242 | TS | 0.040091 | TCIG2 | 0.028436 |
| BIB | 0.030238 | TCIG3 | 0.035513 | IBB | 0.024543 |
| VBI | 0.029938 | UM | 0.030291 | BMB | 0.021986 |
| VPBI | 0.02973 | TCIG2 | 0.029418 | UM | 0.020226 |
| TSBME | 0.025687 | TCBE | 0.026958 | BBI | 0.018173 |
| TS | 0.02038 | LCBE | 0.025977 | VBI | 0.017993 |
| LCBE | 0.01966 | VBMI | 0.017602 | TSBME | 0.017798 |
| TCBE | 0.019331 | VPBMI | 0.017342 | VPBI | 0.011275 |
| TCIG3 | 0.017419 | VBI | 0.013967 | VBM | 0.009648 |
| VBM | 0.013918 | VPBI | 0.013633 | TCBE | 0.009475 |
| TCIG2 | 0.012677 | BMB | 0.012045 | VBMI | 0.008978 |
| VPBM | 0.009456 | IBB | 0.011377 | MBB | 0.008135 |
| VBMI | 0.006269 | VPBM | 0.009934 | BBM | 0.007122 |
| VPBMI | 0.005409 | BBI | 0.008782 | TS | 0.007066 |
| IBB | 0.005153 | VBM | 0.008439 | VPBMI | 0.006914 |
| BBI | 0.00344 | MBB | 0.00652 | VPBM | 0.006676 |
| BMB | 0.003377 | BBM | 0.005116 | VPB | 0.006059 |
| MBB | 0.002158 | VPB | 0.00024 | VB | 0.005058 |
| BBM | 0.001635 | VB | 0.000138 | TSBE | 0.003802 |

TABLE 4: Feature importance table for classifier 1 based on the random forest classifier along with their importance score for classifier 1, for sentence level classification.

The feature importance scores for each feature corresponding to each classification can be used as a metric for building a decision tree. It can also be used as a decision metric to design a new classifier either as a subset of the existing features or in combination with new/other features. The features and the feature importance values can help in document classification, document clustering, automatic question answer generation as well as other methods of relationship extraction from biomedical text.

**Creation of Classifier 2 for 'readability classification'**

A set of biomedical text abstracts were manually annotated for readability into two bins namely 'easy to read' (count = 100) and 'difficult to read' (count = 100) by a group of annotators (working in the domain of microbiome). The assignment of an abstract as 'easy to read' or 'difficult to read' was decided based on the majority voting by the group. Abstracts with a tie were discarded. This set was used for training and validation of the classifier 2. The same set of 29 domain features were used to train the classifier 2. A classification ('classifier 2') was performed using four algorithms namely Naive Bayes, Logistic Regression, SVM and Random Forests. Optimum hyper-parameters for SVM and Random Forest classifiers are obtained using Grid Searching. For Bag-of-words and TF-iDF models, top 100 features were considered with up to 4-grams. The algorithms were trained on 90% samples and tested on 10% chosen randomly. Re-sampling techniques were implemented using the 'imbalanced-learn' python package to minimize biases arising due to imbalanced classes. Optimum parameter estimation is done by 5-fold cross validated grid searching on a predefined parameter grid. During each parameter search operation, the training samples are split into 5 segments, wherein 4 are used for training and 1 for validation. However, as this classification takes a random set of training and test data from the main corpus, a 100-step bootstrap validation was performed for all the classifiers in order to avoid biases in the result. For every bootstrap step, a random 90% of samples were chosen as a training set and a random 10% as a test set. The mean values of the bootstrapped output are tabulated below in TABLE 5.

TABLE 5: Comparative performance of classifier 2 shows that the set of domain features outperform other generic feature for readability classifiers. BOW denotes Bag of Words features, TFIDF corresponds to TDIDF features.

| BOW | Precision | Recall | F1 Score | Accuracy |
|---|---|---|---|---|
| Naive Bayes | 0.631931 | 0.609 | 0.607627 | 0.609 |
| Logistic Regression | 0.701685 | 0.68 | 0.680243 | 0.68 |
| SVM | 0.746606 | 0.72 | 0.719712 | 0.72 |
| Random Forest | 0.656942 | 0.627 | 0.627477 | 0.627 |
| | | | | |
| TFIDF | Precision | Recall | F1 Score | Accuracy |
| Naive Bayes | 0.501955 | 0.479 | 0.480756 | 0.479 |
| Logistic Regression | 0.537172 | 0.509 | 0.508485 | 0.509 |
| SVM | 0.485293 | 0.473 | 0.456426 | 0.473 |
| Random Forest | 0.562934 | 0.5355 | 0.535263 | 0.5355 |
| | | | | |
| DOMAIN features | Precision | Recall | F1 Score | Accuracy |
| Naive Bayes | 0.393903 | 0.51 | 0.373769 | 0.51 |
| Logistic Regression | 0.769377 | 0.74 | 0.739734 | 0.74 |
| SVM | 0.795633 | 0.77 | 0.7693 | 0.77 |
| Random Forest | 0.770806 | 0.745 | 0.745008 | 0.745 |

As can be seen from the above results, the set of domain features could also distinguish the readability of the biomedical text having bacterial associations with an overall higher accuracy (including the Precision, Recall and F1 score) especially using the Logistic Regression, SVM and Random Forest classifier. Similar to Classifier 1, in order to estimate the individual feature's contribution to the classifier, a feature importance score is computed for each feature with a higher score denoting higher importance as shown in TABLE 6. Feature importance for this case was done for Random Forest classifier using Gini index measure as described earlier. A feature is given a higher importance, if its elimination from the feature set causes the Gini score of the data to increase. Importance scores (as calculated for the features in TABLE 6) are therefore normalized total reduction in Gini score due the absence of that feature. Higher the importance score, more importance does a feature hold for the corresponding classification. In simple words, a feature is deemed important, if its presence increases the information about the sample. For example, if the presence of interaction keywords is considered as a feature, then it is safe to conclude that the presence of this feature in any sentence should increase the chances of a reported interaction in that sentence. In the decision tree, the aim of every split is to decrease the Gini score of the subsets. A branch stops splitting further if its Gini score =0, or in other words, it has items from a single class only. A feature's importance can thus be estimated by the fraction of Gini score lost when that feature is eliminated from the tree. In a trained random forest, feature importance is the reduction in Gini score due to the absence of that feature, averaged over all the trees in the forest.

TABLE 6: Feature importance table for classifier 2 based on the random forest classifier along with their importance score for classifier 2. Importance scores are therefore normalized total reduction in Gini score due the absence of that feature. Feature importance reported here are averaged across 100 bootstrap iterations.

| Feature | Importance |
| --- | --- |
| TS | 0.220395 |
| TBE | 0.064686 |
| TSBE | 0.061394 |
| BBdist | 0.060776 |
| UB | 0.056698 |
| TIE | 0.055739 |
| TME | 0.054819 |
| UI | 0.046518 |
| TCIG1 | 0.042117 |
| UM | 0.041852 |
| LCBE | 0.040147 |
| TCIG2 | 0.037756 |
| TCBE | 0.032698 |
| TSBME | 0.032209 |
| TCIG3 | 0.031974 |
| IBB | 0.015038 |
| BIB | 0.013986 |
| BBI | 0.010682 |
| VBMI | 0.010123 |
| BMB | 0.009593 |
| VBM | 0.009113 |
| VPBMI | 0.008679 |
| BBM | 0.008525 |
| VPBM | 0.008473 |
| MBB | 0.008014 |
| VBI | 0.00767 |
| VPBI | 0.007489 |
| VB | 0.001458 |
| VPB | 0.00138 |

The feature importance scores for each feature corresponding to each classification can be used as a metric for building a decision tree. It can also be used as a decision metric to design a new classifier either as a subset of the existing features or as a combination with new/other features. The features and the feature importance values can help in document classification, document clustering, spam detection in crowdsourcing as well as other methods of readability analysis from biomedical text.

All the results presented here are generated on Lenovo Thinkpad E495 machine with AMD Ryzen 5 processor. The algorithms are implemented in Python programming language. The Python version used is Python 3.8. Of note, results may not match exactly when re-implemented on a different machine / Python version / Python library version in future. The algorithms used are stochastic in nature and may also lead to differences in the results. However, best efforts were taken to cover the variability. The implementations were tested on multiple machines and have found the results (superiority of classification F1 score of domain features in abstract, sentence and readability classification) to be true in every case, despite minor fluctuations in the values due to the random selecting for the bootstrap steps. In summary, the first classifier (classifier 1) consists of abstract classifier and sentence classifier. The task of the abstract classifier is to classify a given biomedical corpus (e.g.-list of abstracts) into abstracts having a potential bacterial association or not. Given an input biomedical corpus, this classifier generates an output comprising of a shortlisted abstract corpus having abstracts reporting potential bacterial associations, utilizing the unique domain features. The task of the sentence classifier is to classify a given sentence corpus (list of tokenized sentences) into sentences having a potential bacterial association or not. Given an input sentence corpus, the classifier generates an output comprising of a shortlisted sentence corpus having sentences reporting potential bacterial associations, based on the identification of the bacteria and interaction entities along with the positive count of the features 16 to 21 for each of the sentence. Depending on the input corpus (which can either be an abstract corpus or a sentence corpus based on the user requirement), the user can use either the abstract classifier or sentence classifier as a part of first classifier. The second classifier (classifier 2) consists of the readability classifier. The task of the readability classifier is to classify a given biomedical corpus (e.g.-list of abstracts) based on their readability. Given an input biomedical corpus, the readability classifier generates an output of classified biomedical corpus comprising abstracts which are categorized into 'Easy' or 'Difficult' to read. This is followed by a step wherein a threshold annotation time range required to annotate each biomedical abstract based on its readability is also estimated.

**Identification of sentences with probable bacterial associations**

A biomedical text corpus classified using 'classifier 1' can be further analyzed to identify sentences with probable bacterial associations applying the following steps. For the text chunks (e.g. biomedical abstracts) classified as 'category 3a' using classifier 1, split the abstracts into the constituent sentences (sentence tokenization) and use the classifier1 trained with a sentence corpus shown to exhibit the maximum accuracy as described in TABLE 3 to identify sentences with potential bacterial associations. Further, for every sentence identified using classifier 1 as 'TRUE' class (sentence with a potential bacterial association), calculate Feature 16-21 along with the occurrence of each of the patterns BBM, BMB, MBB, IBB, BIB, BBI in any sentence of the biomedical text along with the location information of the individual features in the text as indices (i.e. the start and end position of the sentence that contains the feature along with the index position and names of the bacterial, mechanism and interaction keyword entities in the current text where: B is the detected BACTERIAL ENTITY NAME based on DICT_BACT, M is the detected MECHANISM of association entity name based on DICT_MECH, I is the detected INTERACTION keyword based on DICT_INTR). For each text chunk, the list of sentences having a positive nonzero value for the features BBM, BMB, MBB, IBB, BIB, BBI is identified with the index locations and sent as an output. This resultant output list of all sentences from the text corpus constitute the set of sentences with probable bacterial associations. Such sentences can be used for refinement of bacterial associations obtained from other data driven or machine learning approaches for relationship prediction. These set of sentences can also lower the annotator load when used as in input for a sentence annotation system where the identified entity indices can be used for manual or automatic curation. Further, they can also be used for automatic question answer generation which can serve as useful utility for benchmarking crowdsourcing annotations.

According to an embodiment of the disclosure, the steps used in construction and scoring of refined association network are explained as follows:

**Score 1 calculation:** Given the abundance information of two bacteria (x and y) across n samples having means as $\bar{x}$ and $\bar{y}$ respectively, the Pearson correlation between the two bacteria ($r_{xy}$) can be calculated as:

$$r_{xy} = \frac{\sum_{i=1}^{n}(x_i - \bar{x})(y_i - \bar{y})}{\sqrt{\sum_{i=1}^{n}(x_i - \bar{x})^2}\sqrt{\sum_{i=1}^{n}(y_i - \bar{y})^2}}$$ ..................... (2)

The p-value of the correlation (ps1) can be found using the t-distribution or using a Python functions using standard libraries like scipy.stats.pearsonr (available from scipy version 1.8.1). The correlation value ranges from -1 to +1. The absolute value of $r_{xy}$ can be used as score 1 and the sign can be used as a measure of the nature of association. A '+' sign indicates a positive association, and '-' sign denotes a negative association between the two bacteria (x and y). The absolute (or modulus) value of $r_{xy}$ can be used as score 1 which ranges from 0 to 1. This score 1 is calculated for all possible unique pairs of bacteria in a given bacterial abundance matrix. The ps1 for score 1 can then be used to select the subset of edges denoting a statistically significant association and all the identified edges can be combined to create NT1.

_Score 2 calculation:_ For a given list of bacterial associations in form of a query bacterial association network (e.g., in NT1), the experimental evidence of the associations can be calculated using a score to refine it and create a subnetwork that contains only those edges that have valid experimental evidence. This can be done by constructing search queries with each query consisting of the name of the nodes in the query association network in addition to other keyworks relating to the domain of microbiology. The output of the search containing the list of identified biomedical abstracts corresponding to the search queries can be saved in a table of biomedical corpus Cz containing the unique set of biomedical abstracts along with their unique IDs. Following this, the domain features (Feature 1-29) can be calculated for each abstract in corpus Cz and a matrix of domain features M1 for each unique ID is created. Given M1 as input for classifier 1 trained on the abstract training corpus (CAT1, CAT2, CAT3a and CAT3b), the predicted output O1 (table of abstracts with unique IDs classified as CAT3a) can be used to identify the biomedical abstracts that potentially contain bacterial association. O1 is further used to identify all sentences (classified as 'TRUE') using classifier 1 trained on the sentence corpus (containing a set of sentences with a 'TRUE' and 'FALSE' set) in the containing abstracts that contain a bacteria-bacteria association. The output is saved in a new table O2 containing the positively classified sentences along with the unique ID of the source abstract. This set of sentences in O2 can then be used to predict inter bacterial associations (each association representing an edge with the associating bacteria as the connecting nodes) from the available information (using the entity dictionaries and domain features) and construct a new bacterial association network NT2 by combining the edges. In addition, utilizing the corpus Cz and DICT_BACT, presence of bacterial entity information for each abstract can be identified and stored in a new matrix M2 along with the unique IDs corresponding to each abstract. In the next step, the set of edges common between NT1 and NT2 are identified and combined to create a new bacterial association network NT3. Now, utilizing the information available in O1, O2, M2 as well as other tables generated in the process, the following metrics can be obtained for each edge (having bacteria x and y) in NT3:

Cx = count of abstracts in Cz that contain bacterium x
Cy = count of abstracts in Cz that contain bacterium y
C0xy = count of abstracts in Cz that contain neither bacteria x nor bacteria y
C1xy = count of abstracts in Cz that contain both bacteria x and y
C2xy = count of abstracts in Cz that contain both bacteria x and y and identified by classifier 1 to contain an interbacterial association

$$\text{Score } 2 = \frac{c2_{xy}}{c1_{xy}} \dots\dots\dots\dots\dots (3)$$

The probability value (ps2) of the association between bacteria x and bacteria y can further be calculated using a hypergeometric tests like Fisher exact test using Cx, Cy, C0xy and C2xy. Python functions using standard libraries like scipy.stats.fisher_exact (available from scipy version 1.8.1) can be used to perform this calculation. The edge weight of NT3 can be calculated as a function of score 1, score 2, ps1 and ps2 which in simplest form can be a summation of score 1 and score 2. It can be noted that although NT2 is used to refine NT1 and create a first refined association network NT3, it can also be used to create and update a knowledge graph (using the score as well as other resultant data generated using it) of bacterial association that is created by similar networks from multiple experiments.

_Score 3 calculation:_ The list of biomedical abstracts available in O1 can be computationally annotated using the domain features, DICT_BACT, DICT_INT, DICT_MECH and classifier 1 to identify entity locations as well as potential sentences with bacterial associations. However, not all abstracts classified using classifier 1 can be accurate and might contain false positives. Hence, an additional step of refinement of the previously identified bacterial association network can be performed using manual annotation in a crowdsourcing setup. Necessary steps can be used to eliminate spam annotations using features like annotation time and annotator attentiveness. In this section, we discuss about constructing a score for assigning a weight for each edge in NT3 that has multiple abstracts identified by classifier 1 (to contain bacterial association available from the information of bacterial nodes in the edge) as well as annotated by a set of crowd workers in a crowdsourcing setup. In the first step, an intermediate score is assigned to every valid annotator based on their tendency to over-annotator (reporting more associations than ideal) or under-annotate (reporting less associations than ideal). This is an important criterion for biomedical relation annotations as the relations reported between bacteria might not be explicit most of the time in the text, and the annotator needs to use their domain knowledge to decipher the association and make an annotation. For example, let us suppose there is a sentence: *"Escherichia coli* produces a bacteriocin BCTC which has been reported to hinder food uptake by *Clostridium"*. Now, although there is no explicit mention that *Escherichia coli* is associated with *Clostridium,* one can infer quite easily that there must be an association, because of the bacteriocin. Although in the provided example it was quite straightforward to make the inference, in certain cases, it might be a lot more difficult. In addition to this, there may be instances where even valid (non-spam) annotators would differ in the interactions reported by each annotator. Annotators might lie in a spectrum based on their strictness in reporting associations. On one end of the spectrum, there are the strict annotators, i.e., they only report associations if some association is explicitly stated, and on the other end, there are the lenient annotators, who may report associations even if it is slightly hinted. Ideally, a balance between the two extreme cases is desired and therefore, the annotators who would lie somewhere near the middle of the spectrum are more desirable from the point of view of annotation of such biomedical text with bacterial association. Therefore, for every valid annotator, a score is assigned to quantify how far away from the middle of the spectrum each annotator lies. It is pertinent to note that, for every valid annotator, a record is kept for the annotations performed by them. An intermediate score *Sx* is calculated for every annotator, where:

$$Sx = \frac{\sum_{i \in A} |a_i|}{\sum_{j \in A} \frac{\sum_{k \in K_j} |b_j^k|}{|K_j|}} \quad \dots\dots\dots\dots\dots \quad (4)$$

Where:

A is the set of abstracts annotated by annotator *x,*
$|a_i|$ is the number of unique associations reported in abstract i $\in$ A by the annotator x.
$K_j$ is the set of annotators who have annotated the abstract $j \in A$

$\left|b_j^k\right|$ is the number of unique associations reported in abstract *j* by the annotator *k.*

Essentially the term $\frac{\sum_{k \in K} |b_j^k|}{|K|}$ denotes the average number of unique associations reported for the abstract *j.* The score *Sx* therefore quantifies the ratio of the sum of the number of associations reported by the annotator x to the sum of the average number of associations reported by all annotators for all abstracts annotated by x. Now this score is lower bounded by 0 but does not have an upper bound. In order to normalize this score, the *Sx* for every annotator is taken and scaled using standard scaling.

Essentially,

S = {$S_1$, $S_2$, $S_3$, ..., $S_L$} is a set of *Sx* scores for all the *L* valid annotators. Now the normalized score for each annotator is given by:

$$Z_i = \frac{(S_i - \bar{S})}{\sigma_S} \quad \dots\dots\dots\dots\dots \quad (5)$$

where

$\bar{S}$ is the mean of all values in S
$\sigma_S$ is the standard deviation
Using the above formula, we construct the following vector:

$$Z = \{Z_1, Z_2, Z_3, ..., Z_L\} \quad \dots\dots\dots\dots\dots\dots \quad (6)$$

Z is a set of scores for all the L valid annotators.

It is assumed that the distribution of the Z scores will follow the normal distribution following the central limit theorem. The next step is very similar to standard Z test carried out in statistics. However, in this case, instead of using the statistical significance of the test to rule out or accept an alternate hypothesis, we use the statistical significance value to assign a score to each annotator. Therefore, the annotators having a tendency to over-annotate or under-annotate, i.e., farther away from the mean will get a lower score, and the ones who usually report as many associations as the mean tend to get a higher score. Therefore, the annotators having a tendency to over annotate or under annotate will get a lower score and the ones who usually report as many associations as the mean tend to get a higher score.

Now for every annotator, the two-sided statistical significance is calculated, assuming that Z is normally distributed. The P value is noted and assigned to the annotator as the final score.

$$F = \{P_1, P_2, P_3, \dots, P_L\} \dots\dots\dots\dots (7)$$

Where,

$F$ is the set of final scores for all the $L$ valid annotators.
$P_i$ is the P value of the $Z$ test as determined in the previous step.

In the next step, it is intended to score every reported association. Once the scores for every annotator is found, a score is assigned to all their annotations. This is done in the following way: For every abstract $a$ annotated by $K$ valid annotators, for any association ($d$) reported by one of the valid annotators, we score the association d:

$$Sd = \frac{\sum_{k \in K}(F_k * I[d \in D_k^a]) - \sum_{k \in K}(F_k * I[d \notin D_k^a])}{|K|} \dots\dots\dots (8)$$

where I is the indicator function, i.e.

$$I[x] = \begin{cases} 1 \; if \; x \; is \; true \\ 0 \; otherwise \end{cases}$$

and $D_k^a$ is the set of associations reported by the annotator $k$ for the abstract $a$.

The final score $Sd$ will range between -1 and +1, with a lower score indicating that the association carries less confidence and vice versa. For the purpose of creation of the refined association network, $Sd$ can be further range scaled to lie between 0 and 1 using the formula to obtain score 3 using the minimum (min) and maximum (max) value of $Sd$.

$$\text{Score } 3 = \frac{Sd - \min(Sd)}{\max(Sd) - \min(Sd)} = \frac{Sd + 1}{2} \dots\dots (9)$$

**Calculating the final score combining score 1, 2 and 3:** The Final score for assigning a weight for each edge in NT3 post refinement using crowdsourcing to create the second and final refined association network is calculated as score S equal to the summation of products of score 1 with ps1, product of score 2 and ps2 and score 3.

$$S = (score1 * ps1) + (score2 * ps2) + score3 \dots\dots (10)$$

According to an embodiment of the disclosure, the entity relationship prediction using classifier 1 accompanied by machine learning techniques can be explained as follows:

Although the methodology for finding microbial/bacterial associations using manual annotation with or without crowdsourcing is highly accurate, wherein the reported associations are likely to be true, the main disadvantage of such method is the lack of scalability. For any new disease or any new pair of bacteria, new manual annotators would be needed to annotate the associations. An alternative to this step would be to train machine learning algorithms on the abstracts (along with the reported associations) already annotated by valid annotators. In order to do this first, a dataset on which the model will be trained needs to be collected. The procedure for this is detailed in the following steps need to be performed:

1. Whenever any valid annotator annotates a piece of biomedical text, i.e., sentence containing bacterial pairs, the sentence and the bacterial pairs are stored.
2. Pooling the annotations by several annotators, one can get a dataset comprising of sentences and the bacterial pairs interacting in them. In case a single sentence is annotated by more than one annotator, only the annotations (reported associations) by the highest scoring annotator (based on their F scores) would be stored. Therefore, for one sentence, there would only be one annotation.
3. In case no bacterial associations are reported by an annotator, that is also recorded as it serves as negative data

4. Using the bacterial names dictionaries all bacterial mentions in each of the annotated sentences are identified.

5. If the number of bacterial mentions in the sentence is more than 1:

a. A table is created with the number of rows as the number of combinations, and the number of columns as 4.
b. For every row of the table, i.e., for every possible combination of bacterial mentions, it is populated with the sentence in the first column, the bacterial pairs in the second and third columns, and the presence of a reported association in the fourth column. Therefore, if the bacterial pair have a reported association, a Boolean value equal to 'True' is populated in the fourth column, and if there is no reported association, a Boolean 'False' value is populated.

6. The tables for every such annotated sentence in the dataset are concatenated, i.e. joined row wise- such that the number of columns remain constant at 4.
7. The resultant data table serves as the training data for training a machine learning model. The input of the model being the first three rows of the table, i.e., the sentence, the bacterial pairs, and the output/target is the fourth column, i.e., the Boolean variable which records if there is any association reported among the aforementioned bacterial pairs.

Several machine learning architectures can be used for this, including but not limited to transformer-based models, recurrent neural networks and its modifications like LSTMs, GRUs etc. However, since the input is in text form (comprising of strings viz. sentence and bacterial mention terms), they need to be converted to vectorized form prior to being input into any machine learning model. Appropriate methods for converting the text features into numeric features is employed. Here, the method for using BERT, a transformer based pretrained language model can be used which is explained in brief. For each input data, there are 3 texts, one for the sentence, and the others for each of the bacterial mentions / names. These 3 texts/strings are tokenized using BERT Tokenizer and joined together such that separator tokens are used to mark the boundaries between each of these strings. These tokens are essentially the inputs into the BERT transformer model. The output embedding of the [CLS] token of the BERT model is passed through a sigmoid layer. The output of the sigmoid layer is essentially the probability that the specified pair of bacterial mentions have a reported association as per the provided sentence. During training, Cross Entropy Loss (ISBN: 9780262018029) between the predicated probability and the gold truth / target is used as the objective function, which is minimized using backpropagation and gradient descent, thereby updating the parameters of the model, and training it for association prediction. After training of the model is complete, new unseen texts containing bacterial mentions, along with individual pairs of bacterial mentions can be provided to the model, and the model can predict the probability of an association being present among the bacterial pair. If trained with sufficiently large and diverse data, the model may be able to achieve human level performance in annotation accuracy, or at least reduce the annotation load from humans by filtering out texts/sentences and bacterial pairs having low probability of having an association. In an instantiation, the model described above can be augmented using transfer learning. Several datasets with sentences from biomedical literature reporting protein-protein interactions and drug-drug interactions are available. Since these tasks are somewhat similar in principle to the extraction of microbe-microbe associations (including bacteria-bacteria associations) from biomedical text, using transfer learning they can be used to improve the performance of the microbe-microbe association extraction model. This process involves "transferring" the "learning" from one task to another separate task. The steps are as follows:

1. Using the aforementioned BERT model, first, a model can be trained on labelled texts containing protein-protein interaction and drug-drug interaction datasets, using the same principles as stated above.
2. After that, the model can be retrained on the microbe association data that is obtained using crowdsourcing.

It has been shown that such a "transfer learning" pipeline can often improve the performance of the models in the final tasks, which in our case is microbial/bacterial association extraction.

According to an embodiment of the disclosure, the estimation of threshold time required for an annotation based on the predicted readability can be explained as follows:

Crowdsourcing has emerged to become very popular in recent times. Large scale experiments and data collection and annotation exercises are increasingly done using crowdsourcing. However, because of its inherent nature, crowdsourcing has been vulnerable to malicious or spam attacks. Therefore, detecting spam in crowdsourcing exercises is a very important area of research with applications in a wide variety of fields. It was hypothesized that spam annotators in order to maximize their reward given the constraint (time), would not typically spend time on reading the document and annotate them properly, but make random annotations before moving on to the next abstract. Therefore, the time taken by a crowd worker for an annotation can indicate if it was spam or not. In order to find the time ranges, experiments and studies were carried out under controlled settings. A controlled group of 20 scientists and researchers familiar in the field of microbiome science were asked to annotate a selected set of biomedical text abstracts, i.e., select the relationships reported in the abstract. The annotation data viz. the time needed to annotate the abstract, number of relationships reported in the abstract, the length of the abstract in terms of words and sentences were all collected and stored. Simultaneously, the classifier 2 for readability classification using the set of domain features was used to classify the selected set of biomedical text abstracts. It was hypothesized that the abstracts which are difficult to read need longer time to be annotated if done properly and vice versa. Using the data collected during the controlled annotation experiments, a time range needed to properly annotate an abstract depending on the predicted readability of the abstract is computed. Any crowdsourced annotation which falls outside this range can be treated as potential span and sent for further screening. The results show biomedical abstracts predicted as 'Difficult to read' using 'set of domain features' and a Random forest algorithm (as described in 'classifier 2') ranged with a lower range of 60 seconds and a higher range of 208 seconds for annotators to complete the assigned annotation task. Similarly, the biomedical abstracts classified as 'Easy to read' ranged between a 57 seconds to 166 seconds. These values can be used to estimate a threshold annotation time range (T-threshold) with a low (or minimum) and a high (or maximum) time range required to annotate a biomedical abstract based on its readability as predicted by classifier 2 using the described set of domain features. The crowdsourcing annotation system consists of the following tasks assigned to an annotator: Task 1 included identification of sentences in the given biomedical abstract that indicates a probable bacterial association. The sentence could be selected by a text highlighting feature which in turn can be used to capture its actual start and end index in the current text chunk. The annotator can also manually copy and paste the relevant sentences in a provided text box. Task 2 included identification of the possible bacterial, mechanism and interaction entities in each text chunk and assign a relationship between the observed bacterial names visible in the text chunk by human comprehension. The relationship could be identified by selecting the bacterial names, relationship and mechanism from a text dropdown or similar GUI based menu populated automatically in the crowdsourcing annotation system. The annotator can also manually list the observations in a provided text box indicating the exact bacterial entity names, their mechanism and interaction as visible in the text chunk.

According to an embodiment of the disclosure, in contrast to generic non domain features extracted from a biomedical text file, the set of domain features introduced in the present disclosure provides a higher classification accuracy. The biomedical text is automatically tagged with bacterial, mechanism and association entities which can be easily used for predicting the actual associations providing an improved entity recognition. The tagged biomedical text can be used as an input into a crowdsourcing platform with lower annotator workload. The threshold time estimates provide an estimate of the annotation quality and can be used to detect spam annotations in a crowdsourcing setup. A classifier trained with the set of domain features can be used to refine bacterial association networks obtained from microbiome abundance values in experimental data.

According to an embodiment of the disclosure, the three dictionaries are generated as follows:

**First dictionary:** Entity dictionary for various 'Bacteria' (DICT_BACT): In order to capture maximum number of reported bacterial associations from the PubMed abstracts, an extensive keyword list of bacteria was created. The relevant keywords were collected from five sources, namely, NCBI taxonomy, Green Genes, Integrated Microbial Genomes (IMG), Ribosomal Database Project (RPD) and Medical Subheadings (MeSH). The output was then manually curated and refined to fit the dictionary. The generated 'entity' dictionary encapsulated bacterial entities at every taxonomic rank (from phylum to species level), thereby ensuring fetching maximum number of articles on various bacteria (at every taxon level) having reported mechanisms of association.

**Second dictionary:** Dictionary of entities corresponding to 'Mechanisms of bacterial associations' (DICT_MECH): To understand the bacterial communities in an environment (e.g., microbiomes associated with human or soil or water), it is important to know the mechanisms of their complex associations. Bacterial species utilize a plethora of molecular mechanisms which include involvement of their signal molecules as well as secondary metabolites for bringing about complex ecological interactions. Five major categories of mechanisms have been considered in order to build the dictionary of entities corresponding to bacteria's mechanisms of associations. The chosen mechanisms of bacterial associations can be classified under (1) production of certain bacteria derived compounds for their survival; (2) production of bioactive compounds or antibiotics for obtaining competitive advantage over other microbes; (3) bacterial cell-cell interactions in response to external stimuli; (4) production of certain small molecule for providing antagonistic effects against other bacterial species; (5) certain contact dependent mechanisms to bring about cooperation and/or competition in various ecological interactions. In order to survive in various ecological environments, bacteria produce bacteriocins, certain toxins and major anti-microbial compounds (AMPs). Apart from production of bioactive compounds like siderophores which play a crucial role in iron chelation in iron limiting environments, certain bacteria also produce secondary metabolites like polyketides having antibiotic properties. Both these metabolites provide bacteria with a competitive advantage against other microbes in the microbial community for their survival. Quorum Sensing and Biofilm formation, one of the prominent cell-cell interaction mechanisms in bacterial communities, also act in response to signaling molecules like Auto-inducers (AI-2). In order to have antagonistic effects against other bacterial species in a community, certain bacteria also generate small molecule odorous compounds, called microbial Volatile Organic Compounds or mVOCs. Some VOCs are responsible for promoting the growth of neighboring bacteria present in rhizospheres. Yet another mechanism pertains to involvement of their secretion systems. Numerous bacterial associations involve different types of secretion systems (ranging from Type I to Type VI). They function as either growth promotor or inhibitor in order to bring about cooperation and/or competition in various ecological interactions. A detailed list of keywords, consisting of various secondary metabolites and signaling molecules involved in each of the mechanisms mentioned above were generated from utilizing various secondary data sources. These included 'BACTIBASE' Database on different types of bacteriocins produced in bacterial interactions; 'Siderophore Base' Database containing an extensive list of bacterial siderophores involved in bacterial associations (Siderophore Base - The Web Data Base of Microbial Siderophores, n.d.); 'Cluster-Mine360' Database on numerous types of polyketides produced by bacteria; 'mVOC2.0' Database consisting of various classes of small molecule volatiles secreted by different species of bacteria and 'SigMol' Database on different classes of quorum sensing signaling molecules involved during the cell-cell communication mechanism. A combination of keywords like 'Type 3 Secretion Systems', Type **III** Secretion Systems', 'TIIISS', etc., were used to query obtained information pertaining to the 'Secretion systems' mechanism. The resultant output list was then manually curated and refined to fit the dictionary. The mechanism terms of the dictionary can be derived from the indicated databases as well as can be manually identified by a domain expert curation.

**Third dictionary** - Entity dictionary for associations (DICT_INT): A comprehensive dictionary of keywords specifying bacterial associations was created from a large library of biomedical texts. These keywords were further manually curated and categorized at three levels (group 1, group 2 and group 3) depending on the importance in signifying a bacterial association. Group 1 consists of a list of interaction terms that are manually or computationally or in combination identified to be the most important or relevant or appropriate with respect to bacterial associations. Similarly group 2 consists of a list of interaction terms identified to be of medium importance followed by group 3 consisting of interaction terms of least importance. However, it should be noted that this dictionary does not contain terms that are irrelevant. For example, terms in group 3 which are identified to be least important does not mean that they are irrelevant. One way for identification of importance and grouping into category is done by the utilizing the frequency of occurrence of the interaction terms in a selected set of relevant publicly available biomedical corpus (e.g., a manually curated corpus of biomedical abstracts that report bacterial associations). A Part-of-speech (POS) tagging algorithm can be used to extract verbs from such corpus and the extracting the set of uniquely used verbs. A manual curation is done in the next step to eliminate very commonly used verbs that will be identified to have high frequencies. Following this, another manual curation effort is done to extract the set of verbs that indicate inter-bacterial interactions and a final list of interaction terms along with their frequencies is created. In the next step, the final list is sorted in descending order of the frequency values of the interaction terms in the list and frequency values are range scaled between 0 to 1. An interaction term in the list is categorized into group 1 of the scaled frequency value lies between 0.66 to 1 (>= 0.66 and <=1), group 2 if it lies between 0.33 to 0.66 (>= 0.33 and < 0.66) and group 3 if it lies between 0 to 0.33 (> 0 and <0.33). In a separate implementation a different measure scaled between 0 to 1 can be used instead of the range scaled frequency value. Such implementation can include score manually assigned by the annotator independently or in combination with the frequency values. Any other computationally identified importance score of the interaction terms identified using a statistical or machine learning approach is also within the scope of the invention.

The written description describes the subject matter herein to enable any person skilled in the art to make and use the embodiments.

The disclosure herein addresses unresolved problem related to effective utilization of the biomedical abstract. The embodiment thus provides the method and system for annotation and classification of biomedical text having bacterial associations.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computer like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g. using a plurality of CPUs, GPUs etc.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor implemented method (300) for annotation and classification of biomedical text having bacterial associations, the method (300) comprising:

   identifying (302) a disease with known bacterial basis;

   obtaining (306), via one or more hardware processors, bacterial abundance data from samples, corresponding to the disease using an experimental technique, wherein the samples have a microbiological content, wherein the samples were extracted from each individual in a group of patients suffering from the identified disease, and wherein the bacterial abundance data is used to construct a bacterial taxonomic abundance matrix consisting of abundance information of individual bacterial taxon across the group of patients;

   constructing (308), via the one or more hardware processors, a first bacterial association network NT1 using a statistical correlation to find relationships between the bacteria present in the bacterial taxonomic abundance matrix, wherein the first bacterial association network NT1 comprises 'm' number of bacteria as nodes with their relationship as 'e' number of edges and edge weights as an association strength;

   formulating (310), via the one or more hardware processors, a plurality of search queries for each node in the constructed first bacterial association network, wherein each of the plurality of search queries is searched in a biomedical search engine to obtain output tuples as a set of output lists containing a plurality of biomedical texts, wherein each text is identified by an ID ;

   collating (312), via the one or more hardware processors, unique IDs from the set of output lists to form a list of unique IDs;

   obtaining (314), via the one or more hardware processors, the biomedical text corresponding to each unique ID of the list of unique IDs to generate a biomedical text corpus 'Cz' ;

   calculating (316), via the one or more hardware processors, a set of domain features for each abstract present in the biomedical text corpus 'Cz' to generate a feature count matrix with one set of features for each abstracts, wherein the set of domain features are referred from three domain dictionaries, wherein the three domain dictionaries include a first dictionary, a second dictionary, and a third dictionary, wherein

   the first dictionary is a dictionary of terms indicating bacterial named entities,
   a second dictionary is a dictionary of terms indicating bacterial mechanisms of association, and
   a third dictionary is a dictionary of terms indicating bacterial associations grouped into three categories namely group 1, group 2 and group 3, based on their importance;

   applying (318), via the one or more hardware processors, a first classifier to the feature count matrix to obtain a first list of biomedical texts corresponding to each unique ID, wherein the first list of biomedical texts comprising sentences with potential bacterial associations, wherein the sentences having potential bacterial associations is obtained using the first classifier and if a condition is satisfied in the set of features, wherein the first classifier consists of abstract classifier and sentence classifier,

   a task of the abstract classifier is to classify a given biomedical corpus into abstracts having a potential bacterial association or not;
   utilizing, via the one or more hardware processors, sentences having potential bacterial associations and experimental evidence of the bacterial association edge as seen in biomedical literature to create a first refined association network (320), wherein the potential bacterial associations are in a form of the first bacterial association network NT1 as a query bacterial association network, and an accompanying second bacterial association network NT2, further comprising:

identifying sentences with bacterial entities, interactions entities and mechanism entities for the list of biomedical texts, wherein bacterial entities mentioned in the sentences are connected by an edge;

counting a total occurrence of the edge across the biomedical texts in the lists and assign a normalized edge weight;

generating the second bacterial association network NT2 with 'o' number of nodes and 'p' number of edges with the normalized edge weights as identified by a score of experimental evidence of the bacterial association as seen in biomedical literature; and

finding one or more common edges present in the first bacterial association network NT1 and the second bacterial association network NT2 to calculate a refined bacterial association network NT3 with intersection edges having 'q' number of nodes and 'r' number of edges with edge weight as a function of the edge weights of the association networks NT1 and NT2; and

constructing a score for assigning a weight for each edge in the first refined association network NT3 that has multiple abstracts identified by the first classifier; and

identifying bacterial biomarkers and drivers of a disease by comparing the refined bacterial association network for the diseased group of individuals with the refined bacterial association network for a healthy group of individuals, wherein the bacterial biomarkers and drivers of the disease is identified by comparing changes in local graph properties of the nodes in two networks or by comparing changes in first neighbors of the nodes between two network.

2. The processor implemented method (300) of claim 1, further comprising refining the second bacterial association network by modifying the normalized edge weights, wherein the normalized edge weight is a function of a first score, a second score and a third score, wherein,

the first score is a correlation value of abundance count calculated between two bacteria forming a bacterial association edge from a microbiome experiment,

the second score is a score of experimental evidence of the bacterial association as seen in biomedical literature, and

the third score is a score obtained from manual curation of experimental evidence.

3. The processor implemented method (300) of claim 1, further comprising normalizing the extracted sample to remove various sampling and experimental biases using one of a total sum scaling or a percentage normalization.

4. The processor implemented method (300) of claim 1, wherein the bacterial abundance data is obtained using a frequency of mapping of signature genetic elements in the environmental sample.

5. The processor implemented method (300) of claim 1, wherein the set of domain features is calculated from the biomedical corpus comprising of a plurality of compositional and a plurality of context aware features, wherein the plurality of compositional features comprises total and unique entity counts, sentence specific entity counts and entity presence in combination with parts of speeches, and the plurality of context aware features comprises a count of one or more entity patterns in a given order in one or more sentences with or without in combination to the parts of speeches, a sum of word distance between bacterial entities and a size of largest clusters of consecutive occurring bacterial entities.

6. The processor implemented method (300) of claim 1, wherein the condition is a positive nonzero value for the set of features, wherein set of features corresponds to an occurrence of each of the patterns BBM, BMB, MBB, IBB, BIB, BBI in any sentence in the FIXED ORDER of the biomedical text along with the location information of the individual features in the text as indices, wherein B is the detected BACTERIAL ENTITY NAME based on first dictionary, wherein M is the detected MECHANISM of association entity name based on the second dictionary, wherein I is the detected INTERACTION keyword based on the third dictionary.

7. The processor implemented method (300) of claim 1, wherein the feature count matrix is a two dimensional matrix composed of abundance of each feature across each unique ID of the biomedical corpus.

8. The processor implemented method (300) of claim 1, further comprising identifying therapeutic interventions for curing the disease by using the refined association network.

9. The processor implemented method (300) of claim 1, further comprising creating a knowledge graph of bacterial associations pertaining to healthy and disease state using multiple refined association networks obtained from diverse data available from experimental studies and publicly available biomedical literature.

10. A system (100) for annotation and classification of biomedical text having bacterial associations, the system (100) comprises:

a user interface (104);
one or more hardware processors (108);
a memory (110) in communication with the one or more hardware processors, wherein the one or more hardware processors (108) are configured to execute programmed instructions stored in the memory (110), to:

identify a disease with known bacterial basis (DS);
obtain bacterial abundance data from samples, corresponding to the disease using an experimental technique, wherein the samples have a microbiological content, wherein the samples were extracted from each individual in a group of patients suffering from the identified disease, and wherein the bacterial abundance data is used to construct a bacterial taxonomic abundance matrix consisting of abundance information of individual bacterial taxon across the group of patients;
construct a first bacterial association network NT1 using a statistical correlation to find relationships between the bacteria present in the bacterial taxonomic abundance matrix, wherein the first bacterial association network NT1 comprises 'm' number of bacteria as nodes with their relationship as 'e' number of edges and edge weights (as an association strength;
formulate a plurality of search queries for each node in the constructed first bacterial association network, wherein each of the plurality of search queries is searched in a biomedical search engine to obtain output tuples as a set of output lists containing a plurality of biomedical texts, wherein each text is identified by an ID;
collate unique IDs from the set of output lists to form a list of unique IDs;
obtain the biomedical text corresponding to each unique ID of the list of unique IDs to generate a biomedical text corpus 'Cz';
calculate a set of domain features for each abstract present in the biomedical text corpus 'Cz' to generate a feature count matrix with one set of features for each abstracts, wherein the set of domain features are referred from three domain dictionaries, wherein the three domain dictionaries include a first dictionary, a second dictionary, and a third dictionary, wherein
the first dictionary is a dictionary of terms indicating bacterial named entities,
a second dictionary is a dictionary of terms indicating bacterial mechanisms of association, and
a third dictionary is a dictionary of terms indicating bacterial associations grouped into three categories namely group 1, group 2 and group 3, based on their importance;
apply a first classifier to the feature count matrix to obtain a first list of biomedical texts corresponding to each unique ID, wherein the first list of biomedical texts comprising sentences with potential bacterial associations, wherein the sentences having potential bacterial associations is obtained using the first classifier and if a condition is satisfied in the set of features, wherein
the first classifier consists of abstract classifier and sentence classifier,
a task of the abstract classifier is to classify a given biomedical corpus into abstracts having a potential bacterial association or not;
utilize sentences having potential bacterial associations and experimental evidence of the bacterial association edge as seen in biomedical literature to create a first refined association network, wherein the potential bacterial associations are in a form of the first bacterial association network NT1 as a query bacterial association network, and an accompanying second bacterial association network NT2, further comprising:

identifying sentences with bacterial entities, interactions entities and mechanism entities for the list of biomedical texts, wherein bacterial entities mentioned in the sentences are connected by an edge;
counting a total occurrence of the edge across the biomedical texts in the lists and assign a normalized edge weight;
generating the second bacterial association network NT2 with 'o' number of nodes and 'p' number of edges with the normalized edge weights as identified by a score of experimental evidence of the bacterial association as seen in biomedical literature; and

finding one or more common edges present in the first bacterial association network NT1 and the second bacterial association network NT2 to calculate a refined bacterial association network NT3 with intersection edges having 'q' number of nodes and 'r' number of edges with edge weight as a function of the edge weights of the association networks NT1 and NT2; and

constructing a score for assigning a weight for each edge in the first refined association network NT3 that has multiple abstracts identified by the first classifier; and

identify bacterial biomarkers and drivers of a disease by comparing the refined bacterial association network for the diseased group of individuals with the refined bacterial association network for a healthy group of individuals, wherein the bacterial biomarkers and drivers of the disease is identified by comparing changes in local graph properties of the nodes in two networks or by comparing changes in first neighbors of the nodes between two network.

11. One or more non-transitory machine-readable information storage mediums comprising one or more instructions which when executed by one or more hardware processors cause:

identifying a disease with known bacterial basis (DS) ;

obtaining, bacterial abundance data from the samples corresponding to disease using an experimental technique, wherein the samples have a microbiological content, wherein the samples were extracted from each individual in a group of patients suffering from the identified disease, and wherein the bacterial abundance data is used to construct a bacterial taxonomic abundance matrix consisting of abundance information of individual bacterial taxon across the group of patients;

constructing, via the one or more hardware processors, a first bacterial association network NT1 using a statistical correlation to find relationships between the bacteria present in the bacterial taxonomic abundance matrix, wherein the first bacterial association network NT1 comprises 'm' number of bacteria as nodes with their relationship as 'e' number of edges and edge weights as an association strength;

formulating, via the one or more hardware processors, a plurality of search queries for each node in the constructed first bacterial association network, wherein each of the plurality of search queries is searched in a biomedical search engine to obtain output tuples as a set of output lists containing a plurality of biomedical texts, wherein each text is identified by an ID;

collating, via the one or more hardware processors, unique IDs from the set of output lists to form a list of unique IDs;

obtaining, via the one or more hardware processors, the biomedical text corresponding to each unique ID of the list of unique IDs to generate a biomedical text corpus 'Cz';

calculating, via the one or more hardware processors, a set of domain features for each abstract present in the biomedical text corpus 'Cz' to generate a feature count matrix with one set of features for each abstracts, wherein the set of domain features are referred from three domain dictionaries, wherein the three domain dictionaries include a first dictionary, a second dictionary, and a third dictionary, wherein

the first dictionary is a dictionary of terms indicating bacterial named entities,

a second dictionary is a dictionary of terms indicating bacterial mechanisms of association, and

a third dictionary is a dictionary of terms indicating bacterial associations grouped into three categories namely group 1, group 2 and group 3, based on their importance;

applying, via the one or more hardware processors, a first classifier to the feature count matrix to obtain a first list of biomedical texts corresponding to each unique ID, wherein the first list of biomedical texts further comprising sentences with potential bacterial associations, wherein the sentences having potential bacterial associations is obtained using the first classifier and if a condition is satisfied in the set of features, wherein

the first classifier consists of abstract classifier and sentence classifier,

a task of the abstract classifier is to classify a given biomedical corpus into abstracts having a potential bacterial association or not;

utilizing, via the one or more hardware processors, sentences having potential bacterial associations and experimental evidence of the bacterial association edge as seen in biomedical literature to create a first refined association network, wherein the potential bacterial associations are in a form of the first bacterial association network NT1 as a query bacterial association network, and an accompanying second bacterial association network NT2, further comprising:

identifying sentences with bacterial entities, interactions entities and mechanism entities for the list of biomedical texts, wherein bacterial entities mentioned in the sentences are connected by an edge;

counting a total occurrence of the edge across the biomedical texts in the lists and assign a normalized edge weight;

generating the second bacterial association network NT2 with 'o' number of nodes and 'p' number of edges with the normalized edge weights as identified by a score of experimental evidence of the bacterial association as seen in biomedical literature; and

finding one or more common edges present in the first bacterial association network NT1 and the second bacterial association network NT2 to calculate a refined bacterial association network NT3 with intersection edges having 'q' number of nodes and 'r' number of edges with edge weight as a function of the edge weights of the association networks NT1 and NT2; and

constructing a score for assigning a weight for each edge in the first refined association network NT3 that has multiple abstracts identified by the first classifier; and

identifying bacterial biomarkers and drivers of a disease by comparing the refined bacterial association network for the diseased group of individuals with the refined bacterial association network for a healthy group of individuals, wherein the bacterial biomarkers and drivers of the disease is identified by comparing changes in local graph properties of the nodes in two networks or by comparing changes in first neighbors of the nodes between two network.

**12.** The processor implemented method (300) of claim 1, further comprising:
obtain output list of all sentences from the text corpus constitute the set of sentences describing probable bacterial associations, wherein the set of sentences is used to:

refinement of bacterial associations obtained from data driven or machine learning approaches for relationship prediction,

lower the annotator load, when used as in input for a sentence annotation system where the identified entity indices is used for manual or automatic curation; and

automatic question answer generation which serve as useful utility for benchmarking crowdsourcing annotations.

## Patentansprüche

**1.** Prozessorimplementiertes Verfahren (300) zur Annotation und Klassifizierung von biomedizinischem Text mit bakteriellen Assoziationen, wobei das Verfahren (300) Folgendes umfasst:

Identifizieren (302) einer Krankheit mit bekannter bakterieller Basis;

Erhalten (306), über einen oder mehrere Hardwareprozessoren, von bakteriellen Häufigkeitsdaten aus Proben, die der Krankheit entsprechen, unter Verwendung einer experimentellen Technik, wobei die Proben einen mikrobiologischen Gehalt aufweisen, wobei die Proben von jedem Individuum in einer Gruppe von Patienten, die an der identifizierten Krankheit leiden, extrahiert wurden, und wobei die bakteriellen Häufigkeitsdaten verwendet werden, um eine bakterielle taxonomische Häufigkeitsmatrix zu konstruieren, die aus Häufigkeitsinformationen einzelner bakterieller Taxone über die Gruppe von Patienten besteht;

Konstruieren (308), über den einen oder die mehreren Hardwareprozessoren, eines ersten bakteriellen Assoziationsnetzwerks NT1 unter Verwendung einer statistischen Korrelation, um Beziehungen zwischen den Bakterien zu finden, die in der bakteriellen taxonomischen Häufigkeitsmatrix vorhanden sind, wobei das erste bakterielle Assoziationsnetzwerk NT1 eine "m"-Anzahl von Bakterien als Knoten mit ihrer Beziehung als "e"-Anzahl von Kanten und Kantengewichten als Assoziationsstärke umfasst;

Formulieren (310), über den einen oder die mehreren Hardwareprozessoren, einer Mehrzahl von Suchabfragen für jeden Knoten in dem konstruierten ersten bakteriellen Assoziationsnetzwerk, wobei jede der Mehrzahl von Suchabfragen in einer biomedizinischen Suchmaschine durchsucht wird, um Ausgabetupel als einen Satz von Ausgabelisten zu erhalten, die eine Mehrzahl von biomedizinischen Texten enthalten, wobei jeder Text durch ID identifiziert wird;

Zusammenstellen (312), über den einen oder die mehreren Hardwareprozessoren, eindeutiger IDs aus dem Satz von Ausgabelisten, um eine Liste eindeutiger IDs zu bilden;

Erhalten (314), über den einen oder die mehreren Hardwareprozessoren, des biomedizinischen Textes, der jeder eindeutigen ID der Liste eindeutiger IDs entspricht, um einen biomedizinischen Textkorpus "Cz" zu erzeugen;

Berechnen (316), über den einen oder die mehreren Hardwareprozessoren, eines Satzes von Domänenmerkmalen für jede Abstraktion, die in dem biomedizinischen Textkorpus "Cz" vorhanden ist, um eine Merkmalszählmatrix mit einem Satz von Merkmalen für jede Abstraktion zu erzeugen, wobei auf den Satz von Domänenmerkmalen aus drei Domänenwörterbüchern verwiesen wird, wobei die drei Domänenwörterbücher ein erstes Wörterbuch, ein zweites Wörterbuch und ein drittes Wörterbuch beinhalten, wobei

das erste Wörterbuch ein Wörterbuch von Begriffen ist, die bakterielle benannte Entitäten angeben,

ein zweites Wörterbuch ein Wörterbuch von Begriffen ist, die bakterielle Assoziationsmechanismen angeben, und

ein drittes Wörterbuch ein Wörterbuch von Begriffen ist, die bakterielle Assoziationen angeben, die basierend auf ihrer Wichtigkeit in drei Kategorien, nämlich Gruppe 1, Gruppe 2 und Gruppe 3, gruppiert sind;

Anwenden (318), über den einen oder die mehreren Hardwareprozessoren, eines ersten Klassifizierers auf die Merkmalszählmatrix, um eine erste Liste biomedizinischer Texte zu erhalten, die jeder eindeutigen ID entsprechen, wobei die erste Liste biomedizinischer Texte Sätze mit potentiellen bakteriellen Assoziationen umfasst, wobei die Sätze mit potentiellen bakteriellen Assoziationen unter Verwendung des ersten Klassifizierers erhalten werden, und wenn eine Bedingung in dem Satz von Merkmalen erfüllt ist, wobei

der erste Klassifizierer aus einem abstrakten Klassifizierer und einem Satzklassifizierer besteht,

eine Aufgabe des abstrakten Klassifizierers darin besteht, einen gegebenen biomedizinischen Korpus in Abstrakte zu klassifizieren, die eine potentielle bakterielle Assoziation aufweisen oder nicht;

Verwenden, über den einen oder die mehreren Hardwareprozessoren, von Sätzen mit potentiellen bakteriellen Assoziationen und experimentellen Nachweisen der bakteriellen Assoziationskante, wie in der biomedizinischen Literatur zu sehen, um ein erstes verfeinertes Assoziationsnetzwerk (320) zu erzeugen, wobei die potentiellen bakteriellen Assoziationen in einer Form des ersten bakteriellen Assoziationsnetzwerks NT1 als ein bakterielles Abfrageassoziationsnetzwerk und eines begleitenden zweiten bakteriellen Assoziationsnetzwerks NT2 vorliegen, ferner umfassend:

Identifizieren von Sätzen mit bakteriellen Entitäten, Interaktionsentitäten und Mechanismusentitäten für die Liste biomedizinischer Texte, wobei in den Sätzen erwähnte bakterielle Entitäten durch eine Kante verbunden sind;

Zählen eines Gesamtvorkommens der Kante über die biomedizinischen Texte in den Listen und Zuweisen eines normalisierten Kantengewichts;

Erzeugen des zweiten bakteriellen Assoziationsnetzwerks NT2 mit einer "o"-Anzahl von Knoten und einer "p"-Anzahl von Kanten mit den normalisierten Kantengewichten, wie durch eine Bewertung von experimentellen Nachweisen der bakteriellen Assoziation, wie in der biomedizinischen Literatur zu sehen, identifiziert; und

Finden einer oder mehrerer gemeinsamer Kanten, die in dem ersten bakteriellen Assoziationsnetzwerk NT1 und dem zweiten bakteriellen Assoziationsnetzwerk NT2 vorhanden sind, um ein verfeinertes bakterielles Assoziationsnetzwerk NT3 mit Schnittkanten mit einer "q"-Anzahl von Knoten und einer "r"-Anzahl von Kanten mit Kantengewicht als eine Funktion der Kantengewichte der Assoziationsnetzwerke NT1 und NT2 zu berechnen; und

Konstruieren einer Bewertung zum Zuweisen eines Gewichts für jede Kante in dem ersten verfeinerten Assoziationsnetzwerk NT3, das mehrere durch den ersten Klassifikator identifizierte Abstrakte aufweist; und

Identifizieren bakterieller Biomarker und Treiber einer Krankheit durch Vergleichen des verfeinerten bakteriellen Assoziationsnetzwerks für die erkrankte Gruppe von Individuen mit dem verfeinerten bakteriellen Assoziationsnetzwerk für eine gesunde Gruppe von Individuen, wobei die bakteriellen Biomarker und Treiber der Krankheit durch Vergleichen von Änderungen in lokalen Grapheigenschaften der Knoten in zwei Netzwerken oder durch Vergleichen von Änderungen in ersten Nachbarn der Knoten zwischen zwei Netzwerken identifiziert werden.

2. Prozessorimplementiertes Verfahren (300) nach Anspruch 1, ferner umfassend Verfeinern des zweiten bakteriellen Assoziationsnetzwerks durch Modifizieren der normalisierten Kantengewichte, wobei das normalisierte Kantengewicht eine Funktion einer ersten Bewertung, einer zweiten Bewertung und einer dritten Bewertung ist, wobei

die erste Bewertung ein Korrelationswert einer Häufigkeitszahl ist, die zwischen zwei Bakterien berechnet wird, die eine bakterielle Assoziationskante aus einem Mikrobiomexperiment bilden,

die zweite Bewertung eine Bewertung von experimentellen Nachweisen der bakteriellen Assoziation, wie in der biomedizinischen Literatur zu sehen, ist, und

die dritte Bewertung eine Bewertung ist, die aus manueller Kurierung von experimentellen Nachweisen erhalten wird.

3. Prozessorimplementiertes Verfahren (300) nach Anspruch 1, ferner umfassend Normalisieren der extrahierten Probe, um verschiedene Stichproben und experimentelle Verzerrungen zu entfernen, unter Verwendung einer von einer Gesamtsummenskalierung oder einer prozentualen Normalisierung.

4. Prozessorimplementiertes Verfahren (300) nach Anspruch 1, wobei die bakteriellen Häufigkeitsdaten unter Verwendung einer Häufigkeit der Zuordnung von genetischen Signaturelementen in der Umgebungsprobe erhalten werden.

5. Prozessorimplementiertes Verfahren (300) nach Anspruch 1, wobei der Satz von Domänenmerkmalen aus dem biomedizinischen Korpus berechnet wird, der eine Mehrzahl von zusammengesetzten Merkmalen und eine Mehrzahl von kontextbewussten Merkmalen umfasst, wobei die Mehrzahl von zusammengesetzten Merkmalen Gesamt- und eindeutige Entitätszahlen, satzspezifische Entitätszahlen und Entitätsanwesenheit in Kombination mit Teilen von Sprachen umfasst, und die Mehrzahl von kontextbewussten Merkmalen eine Zahl von einem oder mehreren Entitätsmustern in einer gegebenen Reihenfolge in einem oder mehreren Sätzen mit oder ohne Kombination mit den Teilen von Sprachen, eine Summe von Wortabstand zwischen bakteriellen Entitäten und eine Größe von größten Clustern von aufeinanderfolgenden auftretenden bakteriellen Entitäten umfasst.

6. Prozessorimplementiertes Verfahren (300) nach Anspruch 1, wobei die Bedingung ein positiver Nicht-Null-Wert für den Satz von Merkmalen ist, wobei der Satz von Merkmalen einem Auftreten von jedem der Muster BBM, BMB, MBB, IBB, BIB, BBI in einem beliebigen Satz in der FESTEN REIHENFOLGE des biomedizinischen Textes zusammen mit den Ortsinformationen der einzelnen Merkmale in dem Text als Indizes entspricht, wobei B der erkannte BAKTERIELLE ENTITÄTSNAME basierend auf dem ersten Wörterbuch ist, wobei M der erkannte MECHANISMUS der Assoziationsentitätsname basierend auf dem zweiten Wörterbuch ist, wobei I das erkannte INTERAKTIONSschlüsselwort basierend auf dem dritten Wörterbuch ist.

7. Prozessorimplementiertes Verfahren (300) nach Anspruch 1, wobei die Merkmalszahlmatrix eine zweidimensionale Matrix ist, die aus der Häufigkeit jedes Merkmals über jede eindeutige ID des biomedizinischen Korpus besteht.

8. Prozessorimplementiertes Verfahren (300) nach Anspruch 1, ferner umfassend Identifizieren therapeutischer Eingriffe zur Heilung der Krankheit unter Verwendung des verfeinerten Assoziationsnetzwerks.

9. Prozessorimplementiertes Verfahren (300) nach Anspruch 1, ferner umfassend Erstellen eines Wissensgraphen von bakteriellen Assoziationen, die sich auf einen gesunden und Krankheitszustand beziehen, unter Verwendung mehrerer verfeinerter Assoziationsnetzwerke, die aus diversen Daten erhalten werden, die aus experimentellen Studien und öffentlich verfügbarer biomedizinischer Literatur verfügbar sind.

10. System (100) zur Annotation und Klassifizierung von biomedizinischem Text mit bakteriellen Assoziationen, wobei das System (100) Folgendes umfasst:

eine Benutzerschnittstelle (104);
einen oder mehrere Hardwareprozessoren (108);
einen Speicher (110) in Kommunikation mit dem einen oder den mehreren Hardwareprozessoren, wobei der eine oder die mehreren Hardwareprozessoren (108) konfiguriert sind, um programmierte Anweisungen auszuführen, die in dem Speicher (110) gespeichert sind, um:

eine Krankheit mit bekannter bakterieller Basis (DS) zu identifizieren;
bakterielle Häufigkeitsdaten aus Proben zu erhalten, die der Krankheit entsprechen, unter Verwendung einer experimentellen Technik, wobei die Proben einen mikrobiologischen Gehalt aufweisen, wobei die Proben von jedem Individuum in einer Gruppe von Patienten, die an der identifizierten Krankheit leiden, extrahiert wurden, und wobei die bakteriellen Häufigkeitsdaten verwendet werden, um eine bakterielle taxonomische Häufigkeitsmatrix zu konstruieren, die aus Häufigkeitsinformationen einzelner bakterieller Taxone über die Gruppe von Patienten besteht;
ein erstes bakterielles Assoziationsnetzwerk NT1 unter Verwendung einer statistischen Korrelation zu konstruieren, um Beziehungen zwischen den Bakterien zu finden, die in der bakteriellen taxonomischen Häufigkeitsmatrix vorhanden sind, wobei das erste bakterielle Assoziationsnetzwerk NT1 eine "m"-Anzahl von Bakterien als Knoten mit ihrer Beziehung als "e"-Anzahl von Kanten und Kantengewichten (als Assoziationsstärke) umfasst;
eine Mehrzahl von Suchabfragen für jeden Knoten in dem konstruierten ersten bakteriellen Assoziationsnetzwerk zu formulieren, wobei jede der Mehrzahl von Suchabfragen in einer biomedizinischen Suchmaschine durchsucht wird, um Ausgabetupel als einen Satz von Ausgabelisten zu erhalten, die eine Mehrzahl von biomedizinischen Texten enthalten, wobei jeder Text durch eine ID identifiziert wird;

eindeutige IDs aus dem Satz von Ausgabelisten zusammenzustellen, um eine Liste eindeutiger IDs zu bilden;

den biomedizinischen Text zu erhalten, der jeder eindeutigen ID der Liste eindeutiger IDs entspricht, um einen biomedizinischen Textkorpus "Cz" zu erzeugen;

einen Satz von Domänenmerkmalen für jede Abstraktion zu berechnen, die in dem biomedizinischen Textkorpus "Cz" vorhanden ist, um eine Merkmalszählmatrix mit einem Satz von Merkmalen für jede Abstraktion zu erzeugen, wobei auf den Satz von Domänenmerkmalen aus drei Domänenwörterbüchern verwiesen wird, wobei die drei Domänenwörterbücher ein erstes Wörterbuch, ein zweites Wörterbuch und ein drittes Wörterbuch beinhalten, wobei

das erste Wörterbuch ein Wörterbuch von Begriffen ist, die bakterielle benannte Entitäten angeben,

ein zweites Wörterbuch ein Wörterbuch von Begriffen ist, die bakterielle Assoziationsmechanismen angeben, und

ein drittes Wörterbuch ein Wörterbuch von Begriffen ist, die bakterielle Assoziationen angeben, die basierend auf ihrer Wichtigkeit in drei Kategorien, nämlich Gruppe 1, Gruppe 2 und Gruppe 3, gruppiert sind;

einen ersten Klassifizierer auf die Merkmalszählmatrix anzuwenden, um eine erste Liste biomedizinischer Texte zu erhalten, die jeder eindeutigen ID entsprechen, wobei die erste Liste biomedizinischer Texte Sätze mit potentiellen bakteriellen Assoziationen umfasst, wobei die Sätze mit potentiellen bakteriellen Assoziationen unter Verwendung des ersten Klassifizierers erhalten werden, und wenn eine Bedingung in dem Satz von Merkmalen erfüllt ist, wobei

der erste Klassifizierer aus einem abstrakten Klassifizierer und einem Satzklassifizierer besteht,

eine Aufgabe des abstrakten Klassifizierers darin besteht, einen gegebenen biomedizinischen Korpus in Abstrakte zu klassifizieren, die eine potentielle bakterielle Assoziation aufweisen oder nicht;

Sätze mit potentiellen bakteriellen Assoziationen und experimentellen Nachweisen der bakteriellen Assoziationskante, wie in der biomedizinischen Literatur zu sehen, zu verwenden, um ein erstes verfeinertes Assoziationsnetzwerk zu erzeugen, wobei die potentiellen bakteriellen Assoziationen in einer Form des ersten bakteriellen Assoziationsnetzwerks NT1 als ein bakterielles Abfrageassoziationsnetzwerk und eines begleitenden zweiten bakteriellen Assoziationsnetzwerks NT2 vorliegen, ferner umfassend:

Identifizieren von Sätzen mit bakteriellen Entitäten, Interaktionsentitäten und Mechanismentitäten für die Liste biomedizinischer Texte, wobei in den Sätzen erwähnte bakterielle Entitäten durch eine Kante verbunden sind;

Zählen eines Gesamtvorkommens der Kante über die biomedizinischen Texte in den Listen und Zuweisen eines normalisierten Kantengewichts;

Erzeugen des zweiten bakteriellen Assoziationsnetzwerks NT2 mit einer "o"-Anzahl von Knoten und einer "p"-Anzahl von Kanten mit den normalisierten Kantengewichten, wie durch eine Bewertung von experimentellen Nachweisen der bakteriellen Assoziation, wie in der biomedizinischen Literatur zu sehen, identifiziert; und

Finden einer oder mehrerer gemeinsamer Kanten, die in dem ersten bakteriellen Assoziationsnetzwerk NT1 und dem zweiten bakteriellen Assoziationsnetzwerk NT2 vorhanden sind, um ein verfeinertes bakterielles Assoziationsnetzwerk NT3 mit Schnittkanten mit einer "q"-Anzahl von Knoten und einer "r"-Anzahl von Kanten mit Kantengewicht als eine Funktion der Kantengewichte der Assoziationsnetzwerke NT1 und NT2 zu berechnen; und

Konstruieren einer Bewertung zum Zuweisen eines Gewichts für jede Kante in dem ersten verfeinerten Assoziationsnetzwerk NT3, das mehrere durch den ersten Klassifikator identifizierte Abstrakte aufweist; und

Identifizieren bakterieller Biomarker und Treiber einer Krankheit durch Vergleichen des verfeinerten bakteriellen Assoziationsnetzwerks für die erkrankte Gruppe von Individuen mit dem verfeinerten bakteriellen Assoziationsnetzwerk für eine gesunde Gruppe von Individuen, wobei die bakteriellen Biomarker und Treiber der Krankheit durch Vergleichen von Änderungen in lokalen Grapheigenschaften der Knoten in zwei Netzwerken oder durch Vergleichen von Änderungen in ersten Nachbarn der Knoten zwischen zwei Netzwerken identifiziert werden.

11. Ein oder mehrere nichtflüchtige maschinenlesbare Informationsspeichermedien, die eine oder mehrere Anweisungen umfassen, die, wenn sie durch einen oder mehrere Hardwareprozessoren ausgeführt werden, Folgendes bewirken:

Identifizieren einer Krankheit mit bekannter bakterieller Basis (DS);

Erhalten von bakteriellen Häufigkeitsdaten aus den Proben, die der Krankheit entsprechen, unter Verwendung einer experimentellen Technik, wobei die Proben einen mikrobiologischen Gehalt aufweisen, wobei die Proben von jedem Individuum in einer Gruppe von Patienten, die an der identifizierten Krankheit leiden, extrahiert wurden, und wobei die bakteriellen Häufigkeitsdaten verwendet werden, um eine bakterielle taxonomische Häufigkeitsmatrix zu konstruieren, die aus Häufigkeitsinformationen einzelner bakterieller Taxone über die Gruppe von Patienten besteht;

Konstruieren, über den einen oder die mehreren Hardwareprozessoren, eines ersten bakteriellen Assoziationsnetzwerks NT1 unter Verwendung einer statistischen Korrelation, um Beziehungen zwischen den Bakterien zu finden, die in der bakteriellen taxonomischen Häufigkeitsmatrix vorhanden sind, wobei das erste bakterielle Assoziationsnetzwerk NT1 eine "m"-Anzahl von Bakterien als Knoten mit ihrer Beziehung als "e"-Anzahl von Kanten und Kantengewichten als Assoziationsstärke umfasst;

Formulieren, über den einen oder die mehreren Hardwareprozessoren, einer Mehrzahl von Suchabfragen für jeden Knoten in dem konstruierten ersten bakteriellen Assoziationsnetzwerk, wobei jede der Mehrzahl von Suchabfragen in einer biomedizinischen Suchmaschine durchsucht wird, um Ausgabetupel als einen Satz von Ausgabelisten zu erhalten, die eine Mehrzahl von biomedizinischen Texten enthalten, wobei jeder Text durch eine ID identifiziert wird;

Zusammenstellen, über den einen oder die mehreren Hardwareprozessoren, eindeutiger IDs aus dem Satz von Ausgabelisten, um eine Liste eindeutiger IDs zu bilden;

Erhalten, über den einen oder die mehreren Hardwareprozessoren, des biomedizinischen Textes, der jeder eindeutigen ID der Liste eindeutiger IDs entspricht, um einen biomedizinischen Textkorpus "Cz" zu erzeugen;

Berechnen, über den einen oder die mehreren Hardwareprozessoren, eines Satzes von Domänenmerkmalen für jede Abstraktion, die in dem biomedizinischen Textkorpus "Cz" vorhanden ist, um eine Merkmalszählmatrix mit einem Satz von Merkmalen für jede Abstraktion zu erzeugen, wobei auf den Satz von Domänenmerkmalen aus drei Domänenwörterbüchern verwiesen wird, wobei die drei Domänenwörterbücher ein erstes Wörterbuch, ein zweites Wörterbuch und ein drittes Wörterbuch beinhalten, wobei

das erste Wörterbuch ein Wörterbuch von Begriffen ist, die bakterielle benannte Entitäten angeben,

ein zweites Wörterbuch ein Wörterbuch von Begriffen ist, die bakterielle Assoziationsmechanismen angeben, und

ein drittes Wörterbuch ein Wörterbuch von Begriffen ist, die bakterielle Assoziationen angeben, die basierend auf ihrer Wichtigkeit in drei Kategorien, nämlich Gruppe 1, Gruppe 2 und Gruppe 3, gruppiert sind;

Anwenden, über den einen oder die mehreren Hardwareprozessoren, eines ersten Klassifizierers auf die Merkmalszählmatrix, um eine erste Liste biomedizinischer Texte zu erhalten, die jeder eindeutigen ID entsprechen, wobei die erste Liste biomedizinischer Texte ferner Sätze mit potentiellen bakteriellen Assoziationen umfasst, wobei die Sätze mit potentiellen bakteriellen Assoziationen unter Verwendung des ersten Klassifizierers erhalten werden, und wenn eine Bedingung in dem Satz von Merkmalen erfüllt ist, wobei

der erste Klassifizierer aus einem abstrakten Klassifizierer und einem Satzklassifizierer besteht,

eine Aufgabe des abstrakten Klassifizierers darin besteht, einen gegebenen biomedizinischen Korpus in Abstrakte zu klassifizieren, die eine potentielle bakterielle Assoziation aufweisen oder nicht;

Verwenden, über den einen oder die mehreren Hardwareprozessoren, von Sätzen mit potentiellen bakteriellen Assoziationen und experimentellen Nachweisen der bakteriellen Assoziationskante, wie in der biomedizinischen Literatur zu sehen, um ein erstes verfeinertes Assoziationsnetzwerk zu erzeugen, wobei die potentiellen bakteriellen Assoziationen in einer Form des ersten bakteriellen Assoziationsnetzwerks NT1 als ein bakterielles Abfrageassoziationsnetzwerk und eines begleitenden zweiten bakteriellen Assoziationsnetzwerks NT2 vorliegen, ferner umfassend:

Identifizieren von Sätzen mit bakteriellen Entitäten, Interaktionsentitäten und Mechanismentitäten für die Liste biomedizinischer Texte, wobei in den Sätzen erwähnte bakterielle Entitäten durch eine Kante verbunden sind;

Zählen eines Gesamtvorkommens der Kante über die biomedizinischen Texte in den Listen und Zuweisen eines normalisierten Kantengewichts;

Erzeugen des zweiten bakteriellen Assoziationsnetzwerks NT2 mit einer "o"-Anzahl von Knoten und einer "p"-Anzahl von Kanten mit den normalisierten Kantengewichten, wie durch eine Bewertung von experimentellen Nachweisen der bakteriellen Assoziation, wie in der biomedizinischen Literatur zu sehen, identifiziert; und

Finden einer oder mehrerer gemeinsamer Kanten, die in dem ersten bakteriellen Assoziationsnetzwerk NT1 und dem zweiten bakteriellen Assoziationsnetzwerk NT2 vorhanden sind, um ein verfeinertes bakterielles Assoziationsnetzwerk NT3 mit Schnittkanten mit einer "q"-Anzahl von Knoten und einer "r"-Anzahl von Kanten mit Kantengewicht als eine Funktion der Kantengewichte der Assoziationsnetzwerke NT1 und NT2 zu berechnen; und

Konstruieren einer Bewertung zum Zuweisen eines Gewichts für jede Kante in dem ersten verfeinerten Assoziationsnetzwerk NT3, das mehrere durch den ersten Klassifikator identifizierte Abstrakte aufweist; und Identifizieren bakterieller Biomarker und Treiber einer Krankheit durch Vergleichen des verfeinerten bakteriellen Assoziationsnetzwerks für die erkrankte Gruppe von Individuen mit dem verfeinerten bakteriellen Assoziationsnetzwerk für eine gesunde Gruppe von Individuen, wobei die bakteriellen Biomarker und Treiber der Krankheit durch Vergleichen von Änderungen in lokalen Grapheigenschaften der Knoten in zwei Netzwerken oder durch Vergleichen von Änderungen in ersten Nachbarn der Knoten zwischen zwei Netzwerken identifiziert werden.

12. Prozessorimplementiertes Verfahren (300) nach Anspruch 1, ferner umfassend:

Erhalten einer Ausgabeliste aller Sätze aus dem Textkorpus, die den Satz von Sätzen bilden, die wahrscheinliche bakterielle Assoziationen beschreiben, wobei der Satz von Sätzen verwendet wird zum:

Verfeinern von bakteriellen Assoziationen, die aus datengetriebenen oder maschinellen Lernansätzen zur Beziehungsvorhersage erhalten werden,
Senken der Annotatorlast, wenn sie als Eingabe für ein Satzannotationssystem verwendet wird, wobei die identifizierten Entitätsindizes für manuelle oder automatische Kurierung verwendet werden; und
automatischen Erzeugen von Frageantworten, die als nützliches Hilfsmittel zum Benchmarking von Crowdsourcing-Annotationen dienen.

## Revendications

1. Procédé mis en œuvre par processeur (300) pour l'annotation et la classification de texte biomédical ayant des associations bactériennes, le procédé (300) comprenant :

l'identification (302) d'une maladie avec une base bactérienne connue ;
l'obtention (306), via un ou plusieurs processeurs matériels, de données d'abondance bactérienne à partir d'échantillons correspondant à la maladie en utilisant une technique expérimentale, dans lequel les échantillons ont un contenu microbiologique, dans lequel les échantillons ont été extraits de chaque individu dans un groupe de patients souffrant de la maladie identifiée, et dans lequel les données d'abondance bactérienne sont utilisées pour construire une matrice d'abondance taxonomique bactérienne constituée d'informations d'abondance de taxons bactériens individuels à travers le groupe de patients ;
la construction (308), via les un ou plusieurs processeurs matériels, d'un premier réseau d'association bactérienne NT1 en utilisant une corrélation statistique pour trouver des relations entre les bactéries présentes dans la matrice d'abondance taxonomique bactérienne, dans lequel le premier réseau d'association bactérienne NT1 comprend un nombre « m » de bactéries en tant que noeuds avec leur relation en tant que nombre « e » d'arêtes et des poids d'arête en tant que force d'association ;
la formulation (310), via les un ou plusieurs processeurs matériels, d'une pluralité d'interrogations de recherche pour chaque noeud dans le premier réseau d'association bactérienne construit, dans lequel chacune de la pluralité d'interrogations de recherche est recherchée dans un moteur de recherche biomédical pour obtenir des tuples de sortie en tant qu'ensemble de listes de sortie contenant une pluralité de textes biomédicaux, dans lequel chaque texte est identifié par un ID ;
la collationnement (312), via les un ou plusieurs processeurs matériels, d'ID uniques à partir de l'ensemble de listes de sortie pour former une liste d'ID uniques ;
l'obtention (314), via les un ou plusieurs processeurs matériels, du texte biomédical correspondant à chaque ID unique de la liste d'ID uniques pour générer un corpus de texte biomédical « Cz » ;
le calcul (316), via les un ou plusieurs processeurs matériels, d'un ensemble de caractéristiques de domaine pour chaque résumé présent dans le corpus de texte biomédical « Cz » pour générer une matrice de comptage de caractéristiques avec un ensemble de caractéristiques pour chaque résumé, dans lequel l'ensemble de caractéristiques de domaine est référencé à partir de trois dictionnaires de domaine, dans lequel les trois dictionnaires de domaine incluent un premier dictionnaire, un deuxième dictionnaire, et un troisième dictionnaire, dans lequel

le premier dictionnaire est un dictionnaire de termes indiquant des entités nommées bactériennes,
un deuxième dictionnaire est un dictionnaire de termes indiquant des mécanismes d'association bactérienne, et

un troisième dictionnaire est un dictionnaire de termes indiquant des associations bactériennes groupées en trois catégories, à savoir le groupe 1, le groupe 2 et le groupe 3, sur la base de leur importance ;

l'application (318), via les un ou plusieurs processeurs matériels, d'un premier classificateur à la matrice de comptage de caractéristiques pour obtenir une première liste de textes biomédicaux correspondant à chaque ID unique, dans lequel la première liste de textes biomédicaux comprend des phrases avec des associations bactériennes potentielles, dans lequel les phrases ayant des associations bactériennes potentielles sont obtenues en utilisant le premier classificateur et si une condition est satisfaite dans l'ensemble de caractéristiques, dans lequel

le premier classificateur est constitué d'un classificateur de résumés et d'un classificateur de phrases,

une tâche du classificateur de résumés est de classer un corpus biomédical donné en résumés ayant une association bactérienne potentielle ou non ;

l'utilisation, via les un ou plusieurs processeurs matériels, de phrases ayant des associations bactériennes potentielles et de preuves expérimentales de l'arête d'association bactérienne tel que vu dans la littérature biomédicale pour créer un premier réseau d'association affiné (320), dans lequel les associations bactériennes potentielles sont sous une forme du premier réseau d'association bactérienne NT1 en tant que réseau d'association bactérienne d'interrogation, et d'un second réseau d'association bactérienne d'accompagnement NT2, comprenant en outre :

l'identification de phrases avec des entités bactériennes, des entités d'interactions et des entités de mécanisme pour la liste de textes biomédicaux, dans lequel des entités bactériennes mentionnées dans les phrases sont reliées par une arête ;

le comptage d'une occurrence totale de l'arête à travers les textes biomédicaux dans les listes et l'attribution d'un poids d'arête normalisé ;

la génération du second réseau d'association bactérienne NT2 avec un nombre « o » de noeuds et un nombre « p » d'arêtes avec les poids d'arête normalisés tels qu'identifiés par un score de preuves expérimentales de l'association bactérienne tel que vu dans la littérature biomédicale ; et

la recherche d'une ou plusieurs arêtes communes présentes dans le premier réseau d'association bactérienne NT1 et le second réseau d'association bactérienne NT2 pour calculer un réseau d'association bactérienne affiné NT3 avec des bords d'intersection ayant un nombre « q » de noeuds et un nombre « r » d'arêtes avec un poids d'arête en fonction des poids d'arête des réseaux d'association NT1 et NT2 ; et

la construction d'un score pour attribuer un poids pour chaque arête dans le premier réseau d'association affiné NT3 qui a de multiples résumés identifiés par le premier classificateur ; et

l'identification de biomarqueurs bactériens et de facteurs de déclenchement d'une maladie en comparant le réseau d'association bactérienne affiné pour le groupe d'individus malades avec le réseau d'association bactérienne affiné pour un groupe d'individus sains, dans lequel les biomarqueurs bactériens et les facteurs de déclenchement de la maladie sont identifiés en comparant des changements dans des propriétés de graphe local des noeuds dans deux réseaux ou en comparant des changements dans des premiers voisins des noeuds entre deux réseaux.

2. Procédé mis en œuvre par processeur (300) selon la revendication 1, comprenant en outre l'affinage du second réseau d'association bactérienne en modifiant les poids d'arête normalisés, dans lequel le poids d'arête normalisé est une fonction d'un premier score, d'un deuxième score et d'un troisième score, dans lequel,

le premier score est une valeur de corrélation d'un compte d'abondance calculé entre deux bactéries formant une arête d'association bactérienne à partir d'une expérience de microbiome,

le deuxième score est un score de preuves expérimentales de l'association bactérienne tel que vu dans la littérature biomédicale, et

le troisième score est un score obtenu à partir d'une organisation manuelle de preuves expérimentales.

3. Procédé mis en œuvre par processeur (300) selon la revendication 1, comprenant en outre la normalisation de l'échantillon extrait pour éliminer divers biais d'échantillonnage et expérimentaux en utilisant l'une d'une mise à l'échelle de somme totale ou d'une normalisation en pourcentage.

4. Procédé mis en œuvre par processeur (300) selon la revendication 1, dans lequel les données d'abondance bactérienne sont obtenues en utilisant une fréquence de mappage d'éléments génétiques de signature dans l'échantillon environnemental.

5. Procédé mis en œuvre par processeur (300) selon la revendication 1, dans lequel l'ensemble de caractéristiques de domaine est calculé à partir du corpus biomédical comprenant une pluralité de caractéristiques compositionnelles et une pluralité de caractéristiques sensibles au contexte, dans lequel la pluralité de caractéristiques compositionnelles comprend des comptes d'entités totaux et uniques, des comptes d'entités spécifiques à une phrase et une présence d'entité en combinaison avec des parties de discours, et la pluralité de caractéristiques sensibles au contexte comprend un compte d'un ou plusieurs motifs d'entités dans un ordre donné dans une ou plusieurs phrases avec ou sans combinaison avec les parties de discours, une somme de distance de mot entre des entités bactériennes et une taille de plus grands groupes d'entités bactériennes survenant consécutivement.

6. Procédé mis en œuvre par processeur (300) selon la revendication 1, dans lequel la condition est une valeur positive non nulle pour l'ensemble de caractéristiques, dans lequel l'ensemble de caractéristiques correspond à une occurrence de chacun des motifs BBM, BMB, MBB, IBB, BIB, BBI dans n'importe quelle phrase dans l'ORDRE FIXE du texte biomédical conjointement avec les informations d'emplacement des caractéristiques individuelles dans le texte en tant qu'indices, dans lequel B est le NOM D'ENTITÉ BACTÉRIENNE détecté sur la base du premier dictionnaire, dans lequel M est le MÉCANISME détecté de nom d'entité d'association sur la base du deuxième dictionnaire, dans lequel I est le mot-clé d'INTERACTION détecté sur la base du troisième dictionnaire.

7. Procédé mis en œuvre par processeur (300) selon la revendication 1, dans lequel la matrice de compte de caractéristiques est une matrice bidimensionnelle composée d'une abondance de chaque caractéristique à travers chaque ID unique du corpus biomédical.

8. Procédé mis en œuvre par processeur (300) selon la revendication 1, comprenant en outre l'identification d'interventions thérapeutiques pour guérir la maladie en utilisant le réseau d'association affiné.

9. Procédé mis en œuvre par processeur (300) selon la revendication 1, comprenant en outre la création d'un graphe de connaissances d'associations bactériennes se rapportant à un état sain et de maladie en utilisant de multiples réseaux d'association affinés obtenus à partir de diverses données disponibles à partir d'études expérimentales et de littérature biomédicale accessible au public.

10. Système (100) pour l'annotation et la classification de texte biomédical ayant des associations bactériennes, le système (100) comprenant :

une interface utilisateur (104) ;
un ou plusieurs processeurs matériels (108) ;
une mémoire (110) en communication avec les un ou plusieurs processeurs matériels, dans lequel les un ou plusieurs processeurs matériels (108) sont configurés pour exécuter des instructions programmées stockées dans la mémoire (110), pour :

identifier une maladie avec une base bactérienne connue (DS) ;
obtenir des données d'abondance bactérienne à partir d'échantillons correspondant à la maladie en utilisant une technique expérimentale, dans lequel les échantillons ont un contenu microbiologique, dans lequel les échantillons ont été extraits de chaque individu dans un groupe de patients souffrant de la maladie identifiée, et dans lequel les données d'abondance bactérienne sont utilisées pour construire une matrice d'abondance taxonomique bactérienne constituée d'informations d'abondance de taxons bactériens individuels à travers le groupe de patients ;
construire un premier réseau d'association bactérienne NT1 en utilisant une corrélation statistique pour trouver des relations entre les bactéries présentes dans la matrice d'abondance taxonomique bactérienne, dans lequel le premier réseau d'association bactérienne NT1 comprend un nombre « m » de bactéries en tant que noeuds avec leur relation en tant que nombre « e » d'arêtes et des poids d'arête en tant que force d'association ;
formuler une pluralité d'interrogations de recherche pour chaque noeud dans le premier réseau d'association bactérienne construit, dans lequel chacune de la pluralité d'interrogations de recherche est recherchée dans un moteur de recherche biomédical pour obtenir des tuples de sortie en tant qu'ensemble de listes de sortie contenant une pluralité de textes biomédicaux, dans lequel chaque texte est identifié par un ID ;

collationner des ID uniques à partir de l'ensemble de listes de sortie pour former une liste d'ID uniques ;

obtenir le texte biomédical correspondant à chaque ID unique de la liste d'ID uniques pour générer un corpus de texte biomédical « Cz » ;

calculer un ensemble de caractéristiques de domaine pour chaque résumé présent dans le corpus de texte biomédical « Cz » pour générer une matrice de comptage de caractéristiques avec un ensemble de caractéristiques pour chaque résumé, dans lequel l'ensemble de caractéristiques de domaine est référencé à partir de trois dictionnaires de domaine, dans lequel les trois dictionnaires de domaine incluent un premier dictionnaire, un deuxième dictionnaire, et un troisième dictionnaire, dans lequel

le premier dictionnaire est un dictionnaire de termes indiquant des entités nommées bactériennes,

un deuxième dictionnaire est un dictionnaire de termes indiquant des mécanismes d'association bactérienne, et

un troisième dictionnaire est un dictionnaire de termes indiquant des associations bactériennes groupées en trois catégories, à savoir le groupe 1, le groupe 2 et le groupe 3, sur la base de leur importance ;

appliquer un premier classificateur à la matrice de comptage de caractéristiques pour obtenir une première liste de textes biomédicaux correspondant à chaque ID unique, dans lequel la première liste de textes biomédicaux comprend des phrases avec des associations bactériennes potentielles, dans lequel les phrases ayant des associations bactériennes potentielles sont obtenues en utilisant le premier classificateur et si une condition est satisfaite dans l'ensemble de caractéristiques, dans lequel

le premier classificateur est constitué d'un classificateur de résumés et d'un classificateur de phrases,

une tâche du classificateur de résumés est de classer un corpus biomédical donné en résumés ayant une association bactérienne potentielle ou non ;

utiliser des phrases ayant des associations bactériennes potentielles et des preuves expérimentales de l'arête d'association bactérienne tel que vu dans la littérature biomédicale pour créer un premier réseau d'association affiné, dans lequel les associations bactériennes potentielles sont sous une forme du premier réseau d'association bactérienne NT1 en tant que réseau d'association bactérienne d'interrogation, et d'un second réseau d'association bactérienne d'accompagnement NT2, comprenant en outre :

l'identification de phrases avec des entités bactériennes, des entités d'interactions et des entités de mécanisme pour la liste de textes biomédicaux, dans lequel des entités bactériennes mentionnées dans les phrases sont reliées par une arête ;

le comptage d'une occurrence totale de l'arête à travers les textes biomédicaux dans les listes et l'attribution d'un poids d'arête normalisé ;

la génération du second réseau d'association bactérienne NT2 avec un nombre « o » de noeuds et un nombre « p » d'arêtes avec les poids d'arête normalisés tels qu'identifiés par un score de preuves expérimentales de l'association bactérienne tel que vu dans la littérature biomédicale ; et

la recherche d'une ou plusieurs arêtes communes présentes dans le premier réseau d'association bactérienne NT1 et le second réseau d'association bactérienne NT2 pour calculer un réseau d'association bactérienne affiné NT3 avec des bords d'intersection ayant un nombre « q » de noeuds et un nombre « r » d'arêtes avec un poids d'arête en fonction des poids d'arête des réseaux d'association NT1 et NT2 ; et

la construction d'un score pour attribuer un poids pour chaque arête dans le premier réseau d'association affiné NT3 qui a de multiples résumés identifiés par le premier classificateur ; et

l'identification de biomarqueurs bactériens et de facteurs de déclenchement d'une maladie en comparant le réseau d'association bactérienne affiné pour le groupe d'individus malades avec le réseau d'association bactérienne affiné pour un groupe d'individus sains, dans lequel les biomarqueurs bactériens et les facteurs de déclenchement de la maladie sont identifiés en comparant des changements dans des propriétés de graphe local des noeuds dans deux réseaux ou en comparant des changements dans des premiers voisins des noeuds entre deux réseaux.

**11.** Un ou plusieurs supports de stockage d'informations lisibles par machine non transitoires comprenant une ou plusieurs instructions qui, lorsqu'elles sont exécutées par un ou plusieurs processeurs matériels, amènent :

l'identification d'une maladie avec une base bactérienne connue (DS) ;

l'obtention de données d'abondance bactérienne à partir des échantillons correspondant à la maladie en utilisant une technique expérimentale, dans lequel les échantillons ont un contenu microbiologique, dans lequel les échantillons ont été extraits de chaque individu dans un groupe de patients souffrant de la maladie identifiée, et dans lequel les données d'abondance bactérienne sont utilisées pour construire une matrice d'abondance taxonomique bactérienne constituée d'informations d'abondance de taxons bactériens individuels à travers le groupe de patients ;

la construction, via les un ou plusieurs processeurs matériels, d'un premier réseau d'association bactérienne NT1 en utilisant une corrélation statistique pour trouver des relations entre les bactéries présentes dans la matrice d'abondance taxonomique bactérienne, dans lequel le premier réseau d'association bactérienne NT1 comprend un nombre « m » de bactéries en tant que noeuds avec leur relation en tant que nombre « e » d'arêtes et des poids d'arête en tant que force d'association ;

la formulation, via les un ou plusieurs processeurs matériels, d'une pluralité d'interrogations de recherche pour chaque noeud dans le premier réseau d'association bactérienne construit, dans lequel chacune de la pluralité d'interrogations de recherche est recherchée dans un moteur de recherche biomédical pour obtenir des tuples de sortie en tant qu'ensemble de listes de sortie contenant une pluralité de textes biomédicaux, dans lequel chaque texte est identifié par un ID ;

la collationnement, via les un ou plusieurs processeurs matériels, d'ID uniques à partir de l'ensemble de listes de sortie pour former une liste d'ID uniques ;

l'obtention, via les un ou plusieurs processeurs matériels, du texte biomédical correspondant à chaque ID unique de la liste d'ID uniques pour générer un corpus de texte biomédical « Cz » ;

le calcul, via les un ou plusieurs processeurs matériels, d'un ensemble de caractéristiques de domaine pour chaque résumé présent dans le corpus de texte biomédical « Cz » pour générer une matrice de comptage de caractéristiques avec un ensemble de caractéristiques pour chaque résumé, dans lequel l'ensemble de caractéristiques de domaine est référencé à partir de trois dictionnaires de domaine, dans lequel les trois dictionnaires de domaine incluent un premier dictionnaire, un deuxième dictionnaire, et un troisième dictionnaire, dans lequel

le premier dictionnaire est un dictionnaire de termes indiquant des entités nommées bactériennes,

un deuxième dictionnaire est un dictionnaire de termes indiquant des mécanismes d'association bactérienne, et

un troisième dictionnaire est un dictionnaire de termes indiquant des associations bactériennes groupées en trois catégories, à savoir le groupe 1, le groupe 2 et le groupe 3, sur la base de leur importance ;

l'application, via les un ou plusieurs processeurs matériels, d'un premier classificateur à la matrice de comptage de caractéristiques pour obtenir une première liste de textes biomédicaux correspondant à chaque ID unique, dans lequel la première liste de textes biomédicaux comprend en outre des phrases avec des associations bactériennes potentielles, dans lequel les phrases ayant des associations bactériennes potentielles sont obtenues en utilisant le premier classificateur et si une condition est satisfaite dans l'ensemble de caractéristiques, dans lequel

le premier classificateur est constitué d'un classificateur de résumés et d'un classificateur de phrases, une tâche du classificateur de résumés est de classer un corpus biomédical donné en résumés ayant une association bactérienne potentielle ou non ;

l'utilisation, via les un ou plusieurs processeurs matériels, de phrases ayant des associations bactériennes potentielles et de preuves expérimentales de l'arête d'association bactérienne tel que vu dans la littérature biomédicale pour créer un premier réseau d'association affiné, dans lequel les associations bactériennes potentielles sont sous une forme du premier réseau d'association bactérienne NT1 en tant que réseau d'association bactérienne d'interrogation, et d'un second réseau d'association bactérienne d'accompagnement NT2, comprenant en outre :

l'identification de phrases avec des entités bactériennes, des entités d'interactions et des entités de mécanisme pour la liste de textes biomédicaux, dans lequel des entités bactériennes mentionnées dans les phrases sont reliées par une arête ;

le comptage d'une occurrence totale de l'arête à travers les textes biomédicaux dans les listes et l'attribution d'un poids d'arête normalisé ;

la génération du second réseau d'association bactérienne NT2 avec un nombre « o » de noeuds et un nombre « p » d'arêtes avec les poids d'arête normalisés tels qu'identifiés par un score de preuves expérimentales de l'association bactérienne tel que vu dans la littérature biomédicale ; et

la recherche d'une ou plusieurs arêtes communes présentes dans le premier réseau d'association bactérienne NT1 et le second réseau d'association bactérienne NT2 pour calculer un réseau d'association bactérienne affiné NT3 avec des bords d'intersection ayant un nombre « q » de noeuds et un nombre « r » d'arêtes avec un poids d'arête en fonction des poids d'arête des réseaux d'association NT1 et NT2 ; et

la construction d'un score pour attribuer un poids pour chaque arête dans le premier réseau d'association affiné NT3 qui a de multiples résumés identifiés par le premier classificateur ; et

l'identification de biomarqueurs bactériens et de facteurs de déclenchement d'une maladie en comparant le réseau d'association bactérienne affiné pour le groupe d'individus malades avec le réseau d'association bactérienne affiné pour un groupe d'individus sains, dans lequel les biomarqueurs bactériens et les facteurs de déclenchement de la maladie sont identifiés en comparant des changements dans des propriétés de graphe local des noeuds dans deux réseaux ou en comparant des changements dans des premiers voisins des noeuds entre deux réseaux.

12. Procédé mis en œuvre par processeur (300) selon la revendication 1, comprenant en outre :
l'obtention d'une liste de sortie de toutes les phrases à partir du corpus de texte constituant l'ensemble de phrases décrivant des associations bactériennes probables, dans lequel l'ensemble de phrases est utilisé pour :

l'affinement d'associations bactériennes obtenues à partir d'approches d'apprentissage automatique ou piloté par des données pour une prédiction de relation,
l'abaissement de la charge d'annotateur, lorsqu'elle est utilisée comme entrée pour un système d'annotation de phrase où les indices d'entité identifiés sont utilisés pour une organisation manuelle ou automatique ; et
la génération automatique de réponses à des questions qui servent d'utilité utile pour l'étalonnage comparatif d'annotations de crowdsourcing.

110

102

100

108

Feature
generation unit

114

106

First classifier
generation unit

116

104

Second
classifier
generation unit

118

112

FIG. 1

**FIG. 2**

300

Identify a disease with known bacterial basis ('DS') — 302

Extract a sample having a microbiological content from a group of patients suffering from the identified disease (DS) — 304

Obtain bacterial abundance data from the sample corresponding to the disease using an experimental technique, wherein the bacterial abundance data is used to construct a bacterial taxonomic abundance matrix consisting of abundance information of individual bacterial taxon across the group of patients — 306

Construct a first bacterial association network (NT1) using a statistical correlation to find relationships between the bacteria present in the bacterial taxonomic abundance matrix, wherein the first bacterial association network (NT1) comprises 'm' number of bacteria as nodes (N1, N2,... Nm) with their relationship as 'e' number of edges (E1,E2,....,En) and edge weights (EW1, EW2,..., EWn) as an association strength — 308

Formulate a plurality of search queries for each node in the first bacterial association network, wherein each of the plurality of search queries is searched in a biomedical search engine to obtain output tuples as a set of output lists containing a plurality of biomedical texts, wherein each text is identified by an ID — 310

Collate unique IDs from the set of output lists to form a list of unique IDs — 312

Obtain the biomedical text corresponding to each unique ID of the list of unique IDs to generate a biomedical text corpus 'Cz' — 314

Calculate a set of domain features for each abstract present in the biomedical text corpus 'Cz' to generate a feature count matrix with one set of features for each abstracts — 316

Apply a first classifier to the feature count matrix to obtain a first list of biomedical texts corresponding to each unique ID, wherein the first list of biomedical texts comprising sentences with potential bacterial associations, wherein the sentences having potential bacterial associations is obtained using the first classifier and if a condition is satisfied in the set of features — 318

(A)

**FIG. 3A**

300

Ⓐ

Utilize sentences having potential bacterial associations to create a first refined association network — 320

Apply a second classifier to the feature count matrix corresponding to the first list of biomedical text to obtain a readability for each text in the first list of biomedical text — 322

Estimate a threshold annotation time required to annotate each biomedical text based on its readability — 324

Identify sentences in the first list of biomedical text with probable bacterial associations — 326

Create a table of predicted sentences using the first classifier and calculated domain features for each identified sentences in the first list of biomedical text that contain the bacterial association along with the ID — 328

Record the list of predicted sentences corresponding to the bacterial associations to calculate corresponding count along with their unique IDs — 330

Send the first list of biomedical texts, the estimated threshold annotation time and the recorded list of predicted sentences corresponding to each unique ID, to a crowdsourcing annotation system for improved prediction of bacterial associations — 332

Create a second refined association network utilizing the output of the crowdsourcing annotation system and the first refined association network — 334

**FIG. 3B**

400

Identify patients with disease
of a known bacterial basis

Identify healthy volunteers that
serve as matched controls

Obtain stool samples, extract
genetic material and
construct the bacterial
abundance matrix

Obtain stool samples, extract
genetic material and construct
the bacterial abundance matrix

Construct the disease
bacterial association network

Construct the healthy (or
control) association network

Refine the disease bacterial
association network using the
method described in the
invention

Refine the healthy bacterial
association network using the
method described in the
invention

Compare the changes in local graph properties of the nodes
in the two networks or compare the changes in the first
neighbors of the nodes between two networks to identify
bacterial biomarkers or drivers of the disease

FIG. 4

500

| Identify pathogenic strains in a disease having bacterial basis e.g., pathogenic bacterial strains of Escherichia and Klebsiella in Urogenital infections |

| Identify candidate anti pathogenic probiotic strains from experiments on microbiological samples (e.g., fecal specimens) from healthy human volunteers |

| Prepare a list of the pathogenic bacterial strains to target (pathogen-list) |

| Select a group of bacteria that are known modulators of beneficial metabolites in humans like SCFA (or Short Chain Fatty Acids) using their known functional potential |

| Search a biomedical literature search engine (e.g., PubMed) with at least one pathogen and at least one commensal as a search query. Add an additional filter to search only for 'human' related results |

| Prepare a list of the potential commensal strains of bacteria (commensal-list) |

| Construct a biomedical text corpus corresponding to the search output and generate the novel domain-based feature count matrix using the 29 features for the corpus. Apply classifier 1 as described in the invention using the feature count matrix to obtain the text or list of sentences containing potential bacterial associations. |

| Use the DICT_INT dictionary and the identified sentences using classifier 1 to identify potential commensal bacteria having a competitive or inhibitory relationship over a pathogen. In addition, use the DICT_INT and the identified sentences to identify groups of commensal bacteria having a mutualistic or beneficial relationship among them. |

| Groups of bacteria having a 'negative' (or inhibitory) effect on the pathogen and a 'positive' (or mutualistic) effect among themselves can serve as potential candidates of a probiotic cocktail which can then be sent for experimental validation. |

**FIG. 5**

| DICT_BACTE RIA | DICT_MECH ANISM | DICT_INTER ACTION |
|---|---|---|
| • **Bacterial entities from every taxonomic rank were considered** rather than just species or genus level<br>• Ensures to capture maximum number of bacterial entities in a text | • Mechanisms of bacterial associations were **strategically selected to encompass different niches** like human, marine, soil microbiome, etc., and not limited to a particular niche<br>• Enables extraction of maximum number of bacterial associations in a text | • Relevant keywords pertaining to **nature of bacterial associations** were intelligently collated from a large library of biomedical texts, like 'inhibits', 'promotes', 'reduces', 'activates', etc. were incorporated |

| • Bacterial keywords were fetched from multiple databases<br>• These databases comprises of bacterial names, genomes, including 16S rRNA genes of a wide range of bacterial entities | • Keywords pertaining to bacterial mechanism classes like **bacteriocins, siderophores, polyketides, biofilm and quorum sensing, secretion systems** were fetched from multisource databases<br>• These databases comprises of numerous metabolites/compounds of each mechanism class | • Keywords **categorized into three levels depending on the importance in signifying bacterial association.**<br>• 'Group 1' keywords most likely indicates an association followed by 'Group 2' and 'Group 3' being the set with relatively lower likelihood. |

**Manually curating and shortlisting the relevant keyword entities to fit the dictionaries**

## FIG. 6

47

Query each node of an input
bacterial association network

Biomedical search
engine

Obtain output text corpus

Abstract
classifier

Classified text corpus with
potential bacterial associations

Sentence
tokenize

Sentence corpus

Sentence
classifier

Classified sentences with
potential associations –
'Predicted sentences'

Readability
classifier

List of abstracts along with
information of location of the
predicted sentences

Edges of the input association network
are refined as a function of the three
scores below :
**Score 1** : a value based on correlation of
the microbial pair as seen from
abundance data
**Score 2** : a value based on experimental
evidence of the bacterial association edge
as seen in biomedical literature
**Score 3** : a value obtained from manual
curation of experimental evidence of the
bacterial association edge

Task 1 : Identify sentences from abstract
that indicate association
Task 2 : Identify entities (bacteria,
mechanism and interaction) and their
relationships

Detect spam
annotation
and remove

Crowdsourcing
annotation system

**FIG. 7**

**FIG. 8A**

**FIG. 8A**

**FIG. 8B**

Category 1 :

Category 2 :

Category 3a :

Category 3b :

FIG. 8C

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202121033646